(19) Europäisches Patentamt / European Patent Office / Office européen des brevets

(11) EP 2 088 924 B1

(12) **EUROPEAN PATENT SPECIFICATION**

(45) Date of publication and mention
of the grant of the patent:
**21.10.2020 Bulletin 2020/43**

(21) Application number: **07815566.0**

(22) Date of filing: **15.11.2007**

(51) Int Cl.:
*A61B 5/048* (2006.01)    *A61B 5/00* (2006.01)
*A61B 5/0476* (2006.01)    *A61B 5/0484* (2006.01)

(86) International application number:
**PCT/AU2007/001763**

(87) International publication number:
**WO 2008/061292 (29.05.2008 Gazette 2008/22)**

(54) **NEURODIAGNOSTIC MONITORING AND DISPLAY SYSTEM**

NEURODIAGNOSTISCHES ÜBERWACHUNGS- UND ANZEIGESYSTEM

SYSTÈME DE SURVEILLANCE ET D'AFFICHAGE EN NEURODIAGNOSTIC

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **24.11.2006 AU 2006906589 P**

(43) Date of publication of application:
**19.08.2009 Bulletin 2009/34**

(73) Proprietor: **Cortical Dynamics Limited**
**Western Australia (AU)**

(72) Inventor: **LILEY, David Tibor Julian**
**Yarraville, Victoria 3013 (AU)**

(74) Representative: **Treeby, Philip David William et al**
**Maucher Jenkins**
**26 Caxton Street**
**London SW1H 0RJ (GB)**

(56) References cited:
EP-A1- 1 563 789    EP-A2- 1 704 819
WO-A1-2004/064633    WO-A1-2004/064633
WO-A1-2007/140535    WO-A1-2007/140536

US-A- 5 775 330

• KUIZENGA K. ET LA.: 'Biphasic EEG Changes in Relation to Loss of Consciousness During Induction with Thiopental, Propofol, Etomidate, Midazolam or Sevoflurane' BRITISH JOURNAL OF ANAESTHESIA vol. 86, no. 3, 2001, pages 354 - 360, XP008109637
• REZEK I. ET AL.: 'Depth of Anaesthesia Assessment with Generative Polyspectral Models' PROCEEDINGS OF FOURTH INTERNATIONAL CONFERENCE ON MACHINE LEARNING AND APPLICATIONS (ICMLA'05) 2005, pages 1 - 7, XP010902770
• KRAMER M.A. ET AL.: 'Burification Control of a Seizing Human Cortex' PHYSICAL REVIEW E no. 73, 2006, pages 041928-1 - 041928-15, XP008109639
• KUIZENGA K. ET AL.: 'Quantitative Electroencephalographic Analysis of the Biphasic Concentration-Effect Relationship of Propofol in Surgical Patients During Extradural Analgesia' BRITISH JOURNAL OF ANAESTHESIA vol. 80, 1998, pages 725 - 732, XP008109638
• ROSS M. ET AL.: 'Theoretical Electroencephalogram Stationary Spectrum for a White-Noise-Driven Cortex: Evidence for a General Anesthetic-Induced Phase Transition' PHYSICAL REVIEW E vol. 60, no. 6, PART B, 1999, pages 7299 - 7311, XP008109640

EP 2 088 924 B1

## Description

### FIELD

The present invention relates to a process and system for monitoring brain function based on cortical state and cortical neuronal input.

### BACKGROUND

A process for quantifying brain function may involve analysing the spontaneous or stimulus locked scalp recordable electrical activity from a subject. For example, this includes analysing the waveform of early, middle and/or late stimulus evoked components (e.g. as described in International Patent Publication WO2001/74248); or spectral analysis of spontaneously recorded activity (not in response to a particular or general stimulus) using frequency or time domain methods (e.g. as described in European Patent Application EP0898234); or a hybrid approach in which both spontaneous and evoked EEG activity is analysed to determine brain state (e.g. as described in International Patent Publication WO2004/054441).

While such methods have been shown to have clinical efficacy when appropriately constructed statistical discriminant functions are employed, it is unclear what physiological aspects of behaviour and brain function such measures reflect. For instance, these approaches may be detecting changes in EMG activity, and not EEG activity. The Messner report (published in Anesth Analg, 2003, 97, pp.488-491) describes how the bispectral index declines during neuromuscular blockade in fully awake persons. Recent theoretical and experimental work by Liley et al (as described in International Patent Publication WO2004/064633) proposes a specific theoretical framework that enables the construction of more physiologically specific measures of brain function. This framework enables greater structural and functional specificity in representing changes in cortical activity induced by a variety of internal and external factors.

In assessing a subject's brain function during health, disease and/or therapeutic intervention, it is important to distinguish the factors that give rise to changes in brain function. For example, this includes changes in brain (cortical) state (i.e. the brain's inherent receptivity to input from subcortical or distant cortical sources) and changes in the level of cortical neuronal input (which may occur as a consequence of altered input to the cerebral cortex). While an analysis of the early components of a variety of event related potentials (ERP) may provide information regarding the integrity of the various input pathways to the cortex, this technique is inherently limited as not all cortical areas are the recipient of peripherally derived sensory information. For example, the frontal cortex neither directly nor indirectly (through subcortical nuclei) receives any sensory information. Another limitation of this approach is that, in order to obtain a sufficient signal-to-noise ratio, the evoked response of a number of sequentially presented stimuli must be determined, which clearly limits the temporal resolution of the results obtained. However, there are methods that attempt to improve the temporal resolution by using some form of forecasting method (e.g. as described in International Patent Publication WO2001/74248).

Quantitative EEG (QEEG) methods involving spectral analysis using time or frequency domain methods (e.g. as described in European Patent Application EP0898234) are unable to distinguish between changes in cortical input and brain (cortical) state, because such techniques are unable to make assumptions regarding the physiological sources of changes in EEG spectral power. This is principally a consequence of the heuristic approach of current QEEG methods.

Accordingly, it is difficult to determine whether changes in EEG signals from a subject are caused by changes in cortical input (e.g. to different areas of the brain), or are a consequence of qualitative and quantitative changes in how the cortex responds to this input. There is also no satisfactory way for representing changes in a subject's brain function based on changes in cortical state and the level of cortical neuronal input.

It is desired to address one or more of the above (and particularly the last), or to provide at least a useful alternative.

EP1704819and EP1563789 each disclose a process for analysing an electroencephalogram (EEG) signal representative of activity of a brain of a subject, the former using a histogram transform and the latter using bioimpedance data.

WO2004064633, filed by the present inventor, discloses a method of assessing brain state by analysing EEG readings employing a fixed-order autoregressive moving average (ARMA) filter having autoregressive (AR) order 8and moving average (MA) order 5.

WO2007140535 and WO2007140536, filed by the present inventor but not published before the present filing date, further disclose the above, and also some aspects of the present invention.

### SUMMARY

According to a first aspect of the present invention there is provided a method according to claim 1.

The present invention also provides, in a second aspect, computer executable code stored on computer readable medium to perform any of the steps in the method described above.

The present invention also provides, in a third aspect, a system according to claim 10.

The present invention may be used to assess or monitor a subject's brain function during health, disease or therapeutic intervention.

## BRIEF DESCRIPTION OF THE DRAWINGS

Preferred embodiments of the present invention are herein described, by way of example only, with reference to the accompanying drawings, wherein:

Figure 1 is a block diagram of the components in the EEG processing system;
Figure 2 is a flow diagram of the steps performed under the control of the system;
Figure 3 is an EEG recording interface of the system;
Figure 4 is an EEG recording interface of the system in the review state;
Figure 5 is a sensor diagnostic interface of the system;
Figure 6 is a setup interface of the system;
Figure 7 is a date/time setup interface of the system;
Figure 8 is a system configuration interface of the system;
Figure 9 is an output setup interface of the system;
Figure 10 is an evolving line representation of changes in brain function; and
Figure 11 is a cluster representation of changes in brain function.

## DETAILED DESCRIPTION OF THE PREFERRED EMBODIMENTS

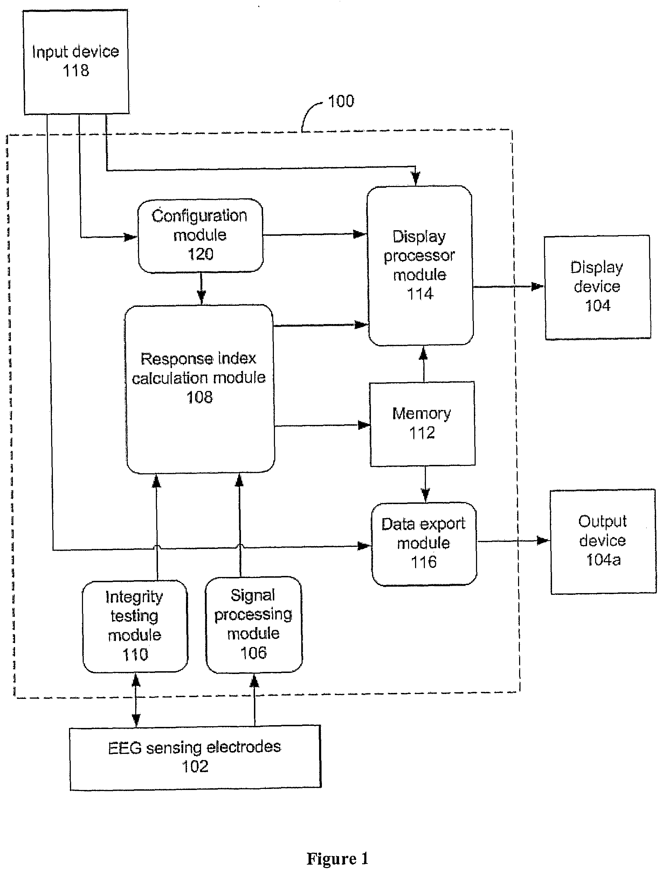

The electroencephalogram (EEG) processing system 100, as shown in Figure 1, includes a signal processing module 106, response index calculation module 108, integrity testing module 110, memory module 112, display processor module 114, data export module 116, and configuration module 120. The modules 106 and 110 are coupled to a by the signal processing module 106 which may compromise the subsequent estimation of the ARMA model coefficients. This involves further removing 50-60 Hz mains contamination using a variety of means or algorithms, such as least mean square adaptive filtering.

The response index calculation module 108 then stores the samples in memory 112 and processes the samples in accordance with a processing option selected by the user. A user may select a processing option that controls the response index calculation module 108 to store samples generated for an EEG recording session, and to retrieve and process the stored samples. The processing performed by module 108 involves generating a plurality of segments, each including a predetermined number of sequential samples (e.g. representative of a 2-second portion of the EEG signal). Module 108 may generate segments based on an incremental (or "sliding window") approach, for example, by generating a new segment at predetermined time intervals so that each new segment includes one or more new samples generated by the signal processing module 106 as well as samples previously generated by the module 106. Module 108 generates, based on the respective samples for each segment, a time invariant autoregressive moving average (ARMA) representation of the EEG signal for each segment (e.g. based on Equation 2). Module 108 then generates brain response data for each segment based on the respective time invariant ARMA representations.

The brain response data for each segment/EEG sample point includes (i) coefficient data representing autoregressive (AR) coefficients and moving average (MA) coefficients; (ii) poles data representing the position of one or more poles on a complex plane determined based on the coefficient data; (iii) zeros data representing the position of one or more zeros on a complex plane determined based on the coefficient data; and (iv) mean pole data representing a mean position of poles determined from the poles data.

The user may select a different processing option that controls the response index calculation module 108 to store the samples in memory 112 and to process the samples based on a recursive approach. The processing performed by module 108 involves generating a time varying ARMA representation of a portion of the EEG signal for each sequential sample point of the EEG signal. A sample point may correspond to each respective sample generated by module 106, or alternatively, module 108 selects new sample points at predetermined time intervals. Module 108 generates coefficient data for each sample point respectively based on a fixed order time varying ARMA representation of the EEG signal that depends on the sampled EEG signal values for the current sample point and for a number of previous sample points, and the coefficient data corresponding to the previous EEG sample point, in a recursive manner (e.g. based on Equation 3). Module 108 then generates poles data, zeros data and/or mean pole data for each sample point based on the corresponding coefficient data for that sample point.

The processing performed by module 108 includes generating AR coefficients and MA coefficients for the ARMA representation for each segment/sample point, and each of the ARMA representations has an AR order of between 8 and 14 and a MA order between 5 and 11. However, the ARMA representation preferably has an AR order of 8 and MA order of 5. The AR and MA coefficients generated for each segment/sample point enables the corresponding ARMA representations (when using the AR and MA coefficients as parameters) to represent the EEG signal for the corresponding segment/sample point.

The samples in each segment represent a different portion of the EEG signal, and the samples in adjacent segments may overlap and represent a common portion of the EEG signal. For example, a segment may include 50% of the samples included in another segment immediately preceding it. The degree of overlap of samples in adjacent segments may vary, and a greater degree of overlap (e.g. having more than 50% of the samples in common) enables better estimation of the AR and MA coefficients. Thus, a more accurate representation of the subject's brain function and/or the level of subcortical input/activity can be provided on the basis of the AR and MA coefficients.

The response index calculation module 108 then generates index data and cortical input data for each segment/sample point based on the corresponding brain response data, and stores the index data and cortical input data in memory 112. The cortical input data represents a product value which represents the level of cortical input to the subject's brain, and which is generated based on the EEG samples, coefficient data, and ARMA representation for the corresponding segment/sample point. The product value may be scaled to fall within a predefined range (e.g. from 0 to 100 inclusive, based on Equations 13 or 14). A larger product value represents a greater level of cortical input to the subject's brain, and a smaller product value represents a lower level of cortical input.

The index data represents an index number that represents the functional state of the subject's brain (i.e. the way in which the brain responds to subcortical input to the brain) and which is generated based on the mean pole data. The index number may be scaled to fall within a predefined range (e.g. from 0 to 100 inclusive, based on Equations 13 or 14). A decrease or inhibition of brain function (e.g. caused by introducing an anaesthetic agent to the subject that decreases cortical response) results in module 108 generating a small index number to represent a lower functional state of the brain. For example, an index number of 0 represents no brain activity. Where brain function is normal or is uninhibited (e.g. during a normal alert state of mind without interventions affecting the cortex), this results in module 108 generating a large index number to represent a higher functional state of the brain. For example, an index number of 100 represents the brain at a fully awake state. Changes in the functional state of the subject's brain can be determined by the changes in the value of the index number for different segments/windows. An advantage of the present invention is that the assessment of brain function of a subject takes into account the degree of brain activity caused by the inherent cortical state.

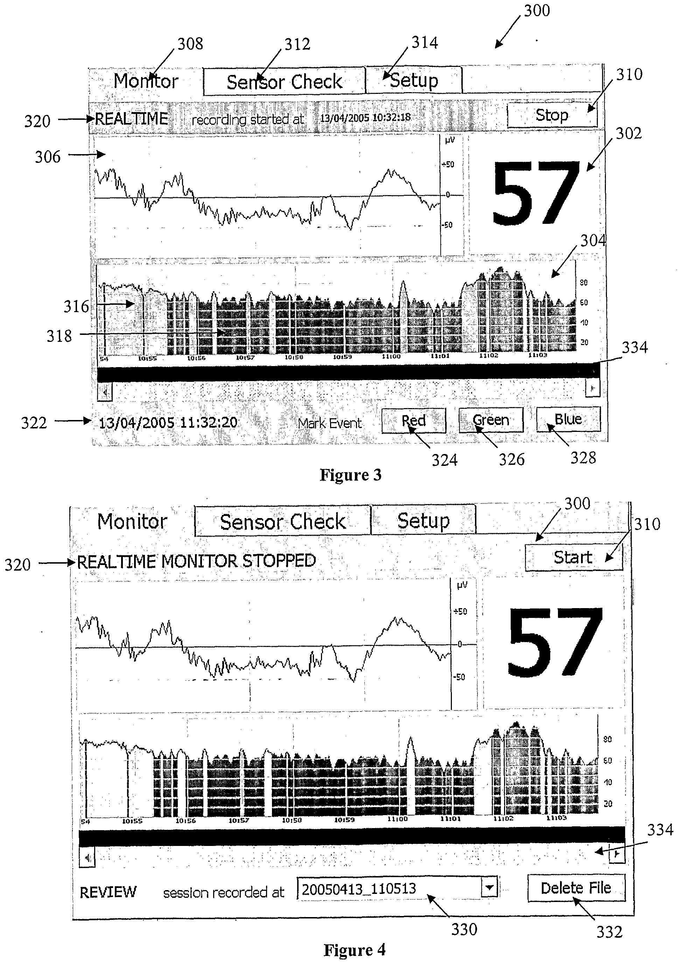

The response index calculation module 108 passes the brain response data, index data and/or cortical input data (collectively referred to as brain state data) to the display processor module 114 for generating display data representing one or more user interface displays for the display device 104 (e.g. a CRT or LCD display). The display processor module. 114 may receive user input from an input device 118 (e.g. a multi-key data entry device or mouse) whilst generating display data for the display device 104. In one embodiment, the input device 118 and display device 104 are combined into one I/O device (e.g. a touch screen display) so that the display processor module 114 receives user input from, and sends display data to, the same I/O device. The display processor module 114 may also generate one or more display interfaces based on the brain response data, index data and/or cortical input data retrieved from memory 112. Figures 3 to 9 are examples of user interface displays generated by module 114.

Figure 3 is an EEG recording interface 300 generated by the display module 114 when the processing an EEG signal using the sliding windows option. The interface 300 includes a monitor tab 308, sensor check tab 312 and setup tab 314 for accessing user interfaces associated with different functions performed by the EEG processing system 100. The interface 300 is generated under the monitor tab 308, and includes a brain response index 302 generated based on the index data, a brain response graph 304 representing changes in the value of the brain response index 302 over time, and an EEG graph 306 representing the detected EEG signal generated based on the EEG samples. The interface 300 includes a control button 310 that enables a user to start and stop an EEG recording/monitoring session performed by the EEG processing system 100. The interface 300 also includes fields for displaying information, such as a date/time field 322 displaying the current date/time, and a status field 320 for displaying the processing option selected by the user and the creation date/time for the record data currently displayed on the interface 300. The interface 300 includes an adjustable scroll bar 334 which enables a user to select a viewing portion of graphs 304 and/or 306 for display on the interface 300.

The interface 300 may include one or more event marker buttons 324, 326, 328 for recording an event associated with each respective button. For example, button 324 may be used for indicating the time at which the subject loses consciousness under anaesthesia, and button 326 may be used for indicating the time at which the subject regains consciousness. Each button 324, 326, 328 is associated with a different colour, and when a button 324, 326, 328 is selected by the user, a line of the corresponding colour is generated on the brain response graph 304 corresponding to the time at which the button was operated. The time positions of events recorded on the brain response graph 304 are stored in memory 112.

The brain response graph 304 of the recording interface 300 is generated based on the brain response index 302 such that a portion of the graph 304 is generated for display in a colour corresponding to a predetermined range of response index 302 values, where each predefined range is represented by a different colour. For example, if the index 302 is between 0 and 20 (inclusive), the corresponding area under the graph 304 is displayed in a first colour (e.g. in blue). If the index 302 is between 21 and 40 (inclusive), the corresponding area under the graph 304 is displayed in a second colour (e.g. in dark green, shown as item 318 in Figure 3). If the index 302 is between 41 and 60 (inclusive), the corresponding area under the graph 304 is displayed in a third colour (e.g. in light green). If the index 302 is between 61 and 80 (inclusive), the corresponding area under the graph 304 is displayed in a fourth colour (e.g. in orange, shown as item 316 in Figure 3). If the index 302 is between 81 and 100 (inclusive), the corresponding area under the graph 304 is displayed in a fifth colour (e.g. in red). The recording interface 300 may include a similar graph generated based on the cortical input data, for example, a portion of the graph is generated for display in a colour corresponding to a predetermined range of product values, where each predefined range is represented by a different colour.

Figure 4 is the EEG recording interface 300 in the review state, i.e. when a user has operated the control button 310 to stop the system 100 from processing EEG signals. As shown in Figure 4, the status field 320 displays a message indicating that processing has stopped. The interface 300 also includes a delete button 332 for deleting data associated with the recent EEG recording from memory 112, and a storage location field 330 (e.g. as a drop down menu) for a user to specify the storage location (e.g. file path and name) and/or parameters for exporting data associated with the recent EEG recording.

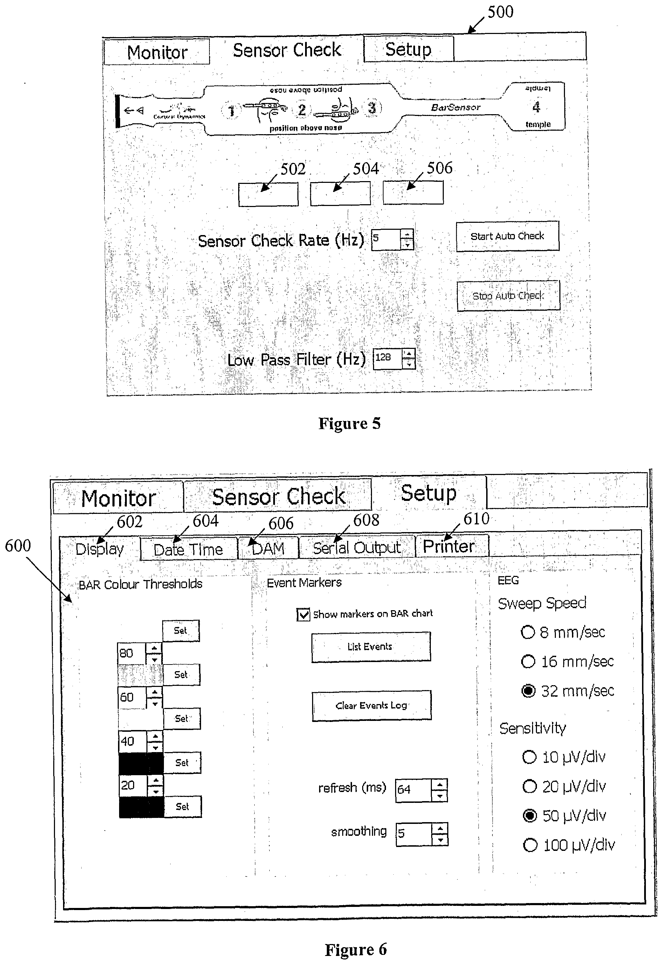

Figure 5 is a sensor diagnostic interface 500 generated by the display module 114 when a user selects the sensor check tab 312. The diagnostic interface 500 enables a user to control a diagnostic process for verifying the operational status of the electrodes 102. The system 100, under the control of the diagnostic process, measures the impedance between each respective electrode and compares it to a reference value. The diagnostic interface 500 includes a flag 502, 504, 506 corresponding to each respective electrode, and a flag for a particular electrode is coloured if the electrode has impedance outside a range (e.g. if it is greater than 5 - 10 kOhms) necessary for accurate performance.

Figure 6 is a setup interface generated by the display module 114 when a user selects the setup tab 314. The setup interface includes a display setup tab 602, date/time setup tab 604, system configuration tab 606, output setup tab 608 and a printer setup tab 610 for accessing user interfaces for configuring operating parameters of the EEG processing system 100. The display module 114 generates a display setup interface 600 when a user selects the display setup tab 602. The interface 600 includes fields for a user to select and/or configure each of the threshold brain response index levels/ranges and their corresponding colours; the events associated with each event marker button 324, 326, 328; the display refresh rate; display smoothing parameters; and the sweep speed and sensitivity (i.e. amplitude) of the brain response graph 304 and/or EEG graph 306.

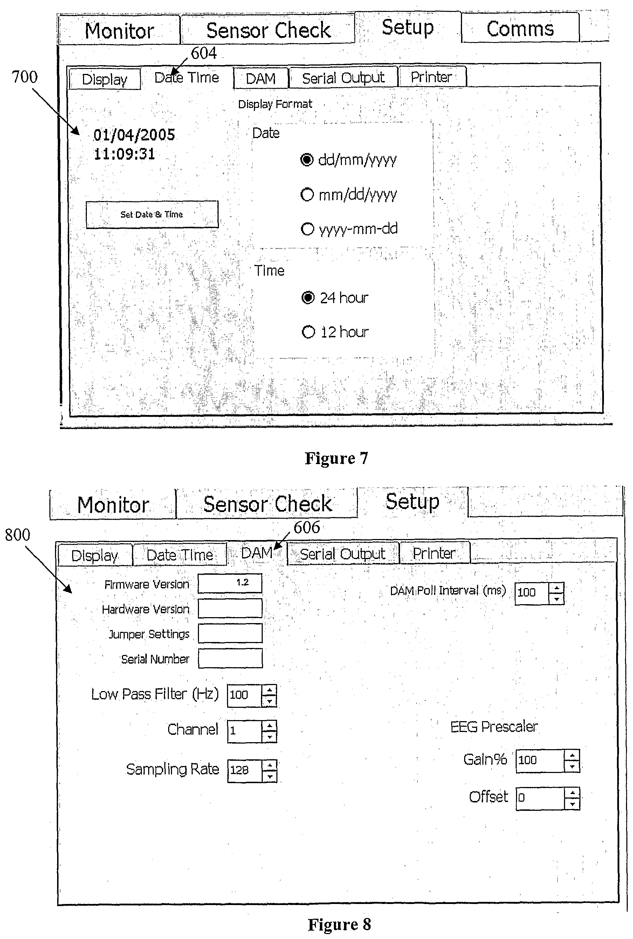

Figure 7 is a date/time setup interface 700 generated by the display module 114 when a user selects the date/time setup tab 604. The interface 700 includes fields for a user to select and/or configure the system clock date/time display format.

Figure 8 is a system configuration interface 800 generated by the display module 114 when a user selects the system configuration tab 606. The interface 800 includes display fields for displaying the serial number, hardware version number, firmware version number, and jumper settings of the system 100. The interface 800 includes fields for a user to select and/or configure parameter values for the low pass filter, the channels for detecting EEG signals, the sampling rate, the polling interval (e.g. in milliseconds), and parameters for prescaling the EEG samples for display on the EEG graph 304.

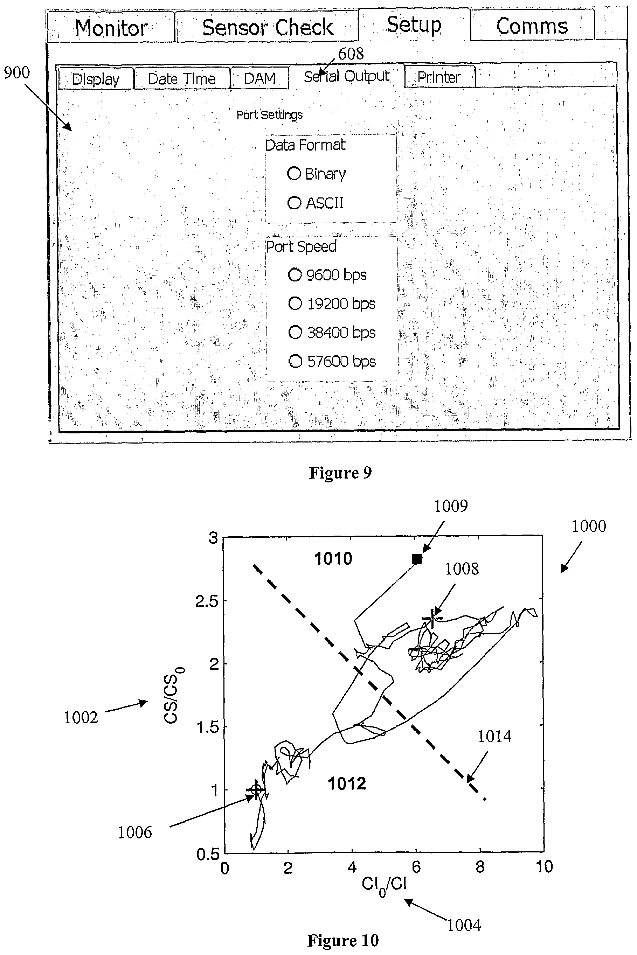

Figure 9 is an output setup interface 900 generated by the display module 114 when a user selects the output setup tab 608. The interface includes fields for a user to select and/or configure the type and/or format of the output data generated by the data export module 116, and the port speed (e.g. for a serial port) of the data export module 116. Output data generated by the data export module 116 is transferred to an output device 104a (e.g. a printer, disk drive, USB port, serial/parallel port, etc.). Output data generated by the data export module 116 may represent:

> i) a patient status report (e.g. including graphs, charts, a summary description of the patient's brain function status, and/or changes of this status over time);
> ii) a signal output representative of a recorded EEG signal; and/or
> iii) a data file including any of the features described above.

The display module 114 generates a printer setup interface when a user selects the printer setup tab 610, which includes user adjustable fields for selecting and configuring the type and/or format of the output data generated by the data export module 116 for an output device 104a (e.g. a printer).

The configuration module 120 of the EEG processing system 100 receives user input from the input device 118, and generates configuration data for controlling the operation of the response index calculation module 108 and the display processor module 114. The configuration data includes control signals, parameters and/or instructions that controls modules 108 and/or 114 to perform one or more of the following:

i) select a processing option for processing the EEG samples;

ii) define the degree of overlap between adjacent segments/windows;

iii) configure module 108 to store the EEG samples, brain response data, index data and/or cortical input data in memory 112;

iv) define the display characteristics of user interfaces generated by the display module (e.g. including the layout of the interface displays, screen size, screen refresh rate, sweep speed and sensitivity of graphs displayed, brain response index threshold ranges and corresponding colours, smoothing settings, etc.)

v) define date and time settings;

vi) define event marker settings (e.g. including the number and type of events associated with each event marker button, and the colour associated with each type of event);

vii) define date export settings (e.g. including the type, format and/or transmission speed of data output to be generated by the data export module 116); and/or

viii) define, select or configure other operation parameters of the system 100 (such as the sensor check rate, and the band-pass filter range (Hz) for the filtering performed by modules 106 and 108).

The integrity testing module 110 continually evaluates the detected EEG signal from the electrodes 102 by comparing the signal against expected parameters (e.g. raw EEG RMS amplitude in the range 2 to 100 microvolts, and 90% of EEG power between 0 and 30 Hz) in order to determine the operational status of the electrodes 102.

An EEG signal detected from a subject can be analysed on the basis of a theoretical assumption that the EEG signal is the result of a filtered random process. As described in International Patent Publication WO2004/064633, an EEG signal detected from a subject could be described mathematically as Equation 1:

$$H_e(\omega;q) = \frac{N(\omega;q)}{D(\omega;q)} P(\omega) \qquad \text{Equation 1}$$

where $H_e$ represents the EEG signal in the frequency domain, and P represents the input into the subject's cortex from other parts of the brain which can be used to assess the functional state of the cortex. N and D define polynomials in $\omega$, the roots of which determine the dominant frequencies of the EEG signal. $P(\omega)$ is assumed to represent Gaussian white noise, and is therefore independent of the frequency $\omega$ (i.e $P(\omega) = P_0$ is a constant that is theoretically determined as being proportional to the magnitude of the subcortical input). In Equation 1, $q$ represents a list of physiological parameters that theoretically determine the coefficients of the polynomials in $\omega$ for both the numerator N and denominator D.

The EEG signal for each respective segment/sample point can be expressed as a respective ARMA time series representation, and more advantageously, as a respective fixed order ARMA time series representation with an autoregressive order of 8 and a moving average order of 5. Equation 2 is a difference equation representing a (8,5) order ARMA representation for generating a representation of a portion of an EEG signal:

$$y[n] = -\sum_{k=1}^{8} a_k y[n-k] + \sum_{k=0}^{5} b_k u[n-k] \qquad \text{Equation 2}$$

where $y[n]$ represents an ordinal sequence of sampled EEG signal values (i.e. $y[n]$ is the $n$-th sequential sample), $y[n-k]$ represents the $k$-th prior sampled value of $y[n]$; $u[n-k]$ represents a Gaussian white noise process; and $a_k$ and $b_k$ is included in the coefficient data and respectively represent the AR (autoregressive) coefficients and MA (moving average) coefficients for a portion of an EEG signal corresponding to a segment. Estimates of the AR and MA coefficients can be generated in a number of ways, for example, using the ARMASA Matlab Toolbox application by P.M.T. Broersen of Delft University of Technology, or using any other ARMA modelling software package.

A time invariant ARMA representation of an EEG signal, as shown in Equation 2 can be re-written as a time varying ARMA time-series representation of an EEG signal as shown in Equation 3:

$$y[n] = -\sum_{k=1}^{8} a_k^{(n)} y[n-k] + \sum_{k=0}^{5} b_k^{(n)} u[n-k] \qquad \text{Equation 3}$$

The AR and MA coefficients for Equation 3, represented by $a_k^{(n)}$ and $b_k^{(n)}$ respectively, are expressed a function of time (for time instant $n$). By denoting Equations 4 and 5:

$$\theta_n = (-a_1^{(n)}, \ldots, -a_8^{(n)}, b_1^{(n)}, \ldots, b_5^{(n)})^T \qquad \text{Equation 4}$$

$$\varphi_n = (y_{n-1}, \ldots, y_{n-8}, u_{n-1}, \ldots, u_{n-5})^T \qquad \text{Equation 5}$$

Equation 3 can be re-written in state-space form as Equation 6:

$$y_n = \varphi_n^T \theta_n + u_n \qquad \text{Equation 6}$$

where $\varphi_n^T$ represents a regression vector, $\theta_n$ represents model parameters (or states) corresponding to those in Equation 4, and $u_n$ represents a Gaussian white noise process corresponding to $u[n-k]$ in Equation 3. By assuming that the model parameters $\theta_n$ evolve as a random walk when no a priori information is available, $\theta_n$ can be estimated recursively from previous values of $\theta_n$ and $y_n$ according to the following general scheme shown in Equation 7:

$$\hat{\theta}_n = \hat{\theta}_{n-1} + K_n \varepsilon_n \qquad \text{Equation 7}$$

where $K_n$ and $\varepsilon_n$ represent the recursively determined filter gain and prediction error of the ARMA model estimated at the previous sample point of the EEG signal, respectively. A variety of methods are available to recursively generate estimates of the time varying AR and MA coefficients $\theta_n$. For example, it is possible to generate coefficient data based on a Kalman adaptive filtering method (e.g. as described in Tarvainen et al, Estimation of non-stationary EEG with Kalman smoother approach: an application to event-related synchronization (ERS), IEEE Trans Biomed Eng, 2004, 51, pp.516-524), or based on any other recursive processing method (e.g. a recursive processing method as described in Ljung L., System Identification - Theory for the User, Prentice Hall, Upper Saddle River, NJ. 2nd edition 1999), or using software (e.g. the functions associated with the MATLAB® System Identification Toolbox version 6.0), to generate optimal estimated values (e.g. in the mean square sense) for the model parameters in $\theta_n$ (expressed as $\hat{\theta}_n$).

Equations 2 and 3 can be rewritten in the z-domain notation, as shown in Equation 8:

$$Y(z) = \frac{\sum_{k=0}^{5} b_k z^{-k}}{\sum_{k=0}^{8} a_k z^{-k}} U(z) \qquad \text{Equation 8}$$

where $Y(z)$ represents an ARMA representation of a portion of the EEG signal in the z-domain; $U(z)$ represents a Gaussian white noise process in the z-domain; and the coefficients $a_k$ and $b_k$ respectively correspond to the AR and MA coefficients for the corresponding segment/sample point. In general, the estimation of ARMA coefficients involves defining $b_0$ and $a_0$ as unity.

The poles associated with the system described by Equation 8 corresponding to the roots of the denominator in Equation 8. The poles data for each segment/sample point are generated based on Equation 9 using the coefficient data for the corresponding segment/sample point (where the poles are represented by $p$). There are 8 possible solutions (or poles) to Equation 9, not all of which are necessarily distinct.

$$\sum_{k=0}^{8} a_k p^{-k} = \sum_{k=0}^{8} a_k p^{8-k} = 0 \qquad \text{Equation 9}$$

The zeros associated with the system described by Equation 8 correspond to the roots of the numerator in Equation 8. The zeros data for each segment/sample point are generated based on Equation 10 using the coefficient data for the corresponding segment/sample point (where the zeros are represented by *z*). There are 5 possible solutions (or zeros) to Equation 10, not all of which are necessarily distinct.

$$\sum_{k=0}^{5} b_k z^{-k} = \sum_{k=0}^{5} b_k z^{5-k} = 0 \qquad \textbf{Equation 10}$$

The poles and zeros represented by the data generated based on Equations 9 and 10 are complex numbers. The poles and zeros for each respective segment/sample point can be plotted on a z-plane, where a change in the position of one or more of the poles and/or zeros, or a change in a mean position of the poles or zeros, represents a change in the functional state of the subject's brain. However, it is technically quite difficult to quantify the functional state of a brain based on the movement of one or more of the poles and/or zeros.

As described in International Patent Publication WO2004/064633, it is expected that various pharmacological interventions result in the motion of a subset of the poles of Equation 8 when plotted in a complex plane (or z-plane). It is possible to quantify the motion of a subset of the poles by generating a value representative of the mean motion of all of the poles represented by the data generated based on Equation 8. In particular, it is found that the mean real part of the pole motion is particularly sensitive to pharmacological manipulation/intervention to a brain.

$$\bar{z}_p \equiv \sum_{i=1}^{i=8} z_{i,p} \qquad \textbf{Equation 11}$$

In Equation 11, $z_{i,p}$ represents the *i*-th pole and thus $\bar{z}_p/8$ represents the mean pole location on a complex plane. Because poles $z_{i,p}$, if complex, exist in complex conjugate pairs $\bar{z}_p/8$ will always be real. Mean pole data is generated based on Equation 12:

$$\bar{z}_p = -a_1 \qquad \textbf{Equation 12}$$

As a consequence of the properties of polynomials, the mean pole location can be determined from the first AR coefficient generated based on Equation 2 (represented as $a_1$) by dividing the negative of the $a_1$ coefficient by 8. However, because the mean pole location is to be scaled to form an appropriate index (i.e. a numeric value that, for example, ranges from 0 to 100) it is not necessary to perform this division to obtain the mean pole location. Instead, it is possible to determine the effect of changes in the mean pole location based on the value of $\bar{z}_p$ itself. Because $\bar{z}_p/8$ will always be greater than or equal to -1 and less than or equal to 1 it can be appropriately scaled so that it extends over the interval 0 to 100. For instance $\bar{z}_p$ may be linearly scaled based on Equation 13 to give an index representative of cortical activity or function:

$$index = c - m\bar{z}_p \qquad \textbf{Equation 13}$$

where *c* and *m* are constants chosen to ensure that index lies in some pre-defined range. $\bar{z}_p$ may also be nonlinearly scaled to give an index representative of cortical activity or function based on Equation 14:

$$index = \frac{d}{1 + e^{-a(\bar{z}_p - b)}} \qquad \textbf{Equation 14}$$

where *a, b* and *d* are constants chosen to ensure that the index lies in some pre-defined range. Index data for each respective segment/sample point represents an index number is generated based on either Equation 13 or Equation 14 using the mean pole data for the corresponding segment/sample point. Because the mean pole motion is inferred to be a measure of changes in the receptivity of the cortex to its incoming input, it is deemed to represent the state of cortex. Therefore in what follows we will refer to this mean pole value, and any corresponding scaling by Equations 13 and 14, as the *cortical state*.

The unscaled mean real pole value represented by the mean pole data for each respective segment/sample point is plotted as a graph to show changes in the unscaled mean real pole value as a function of time or relative to the corresponding segment/sample point number. The unscaled mean real pole is expected to either increase or decrease in response to therapeutic intervention or disease. Alternatively, the mean real pole and the other AR and MA coefficients may be processed by a suitably defined and constructed discriminant function to produce a single scalar quantity representing the functional state of a brain. Such a discriminant function can be determined via stepwise discriminant analysis using any number of commercially available statistical packages, for example Systat (Systat Software Inc, Richmond, USA).

The theoretically derived transfer function shown in Equation 1 can be rewritten in a factored canonical form as Equation 15:

$$H_e(\omega; q) = \frac{g(q) \prod_{k=1}^{k=5}[i\omega - z_k'(q)]}{\prod_{k=1}^{k=8}[i\omega - p_k'(q)]} P(\omega) \qquad \text{Equation 15}$$

where $P(\omega)$ represents the level of cortical input to the brain. Due to the expected temporal complexity of cortical input in the actual cortex, such input is assumed to be indistinguishable from, and representative of, a Gaussian random (white noise) process, i.e $P(\omega) = P_0$. In Equation 15, values for each of the 8 poles (represented by $p_k'$) and 5 zeros (represented by $z_k'$) are determined based on a number of physiological parameters (represented by $q$). The values of $z_k'$ for Equation 15 are generated based on Equation 16 using the zeros data generated based on Equation 10. The values of $p_k'$ for Equation 15 are generated based on Equation 17 using the poles data generated based on Equation 9.

$$z_k' = f_s \ln|z_k| + f_s \text{Arg}(z_k)/2\pi \qquad \text{Equation 16}$$

$$p_k' = f_s \ln|p_k| + f_s \text{Arg}(p_k)/2\pi \qquad \text{Equation 17}$$

where $f_s$ is the EEG sampling (digitisation) frequency. In Equation 15, $g(q)$ represents a gain factor that depends explicitly on one or more of the parameters represented by $q$. In theory, it is expected that the value of $g(q)$ for a subject remains generally unchanged both before and during the application of an intervention to the subject (e.g. an anaesthetic agent) which affects the functional state of the cortex. Accordingly, the value of $g(q)$ is assumed to be a constant. The product $g(q)P(\omega)$ can be used to estimate the level of cortical input to the subject's brain, and since $g(q)$ is assumed to be a constant, it is expected that any changes in the value of $g(q)P(\omega)$ are caused by changes in $P(\omega)$.

The EEG processing system 100 generates, based on Equation 18, cortical input data for representing the cortical neuronal input received by the subject's brain at a particular point in time. Equation 18 assumes that $P(\omega)$ represents Gaussian white noise:

$$g(q)P(\omega) = \frac{\langle \widetilde{Y}(t) \rangle}{\langle Y(t) \rangle} \qquad \text{Equation 18}$$

where $\langle \widetilde{Y}(t) \rangle$ represents an average signal amplitude of a portion of an EEG signal (e.g. of a selected segment of an EEG signal); and $\langle Y(t) \rangle$ represents an ARMA gain value for the corresponding portion of the EEG signal. The value of $\langle \widetilde{Y}(t) \rangle$ can be determined as the root mean square (RMS) of the amplitude of the EEG signal for selected times using a fixed length window, if a time invariant ARMA representation is used, or for every sample point by using a fixed length sliding window, if a time variant ARMA representation is used. The ARMA gain value $\langle Y(t) \rangle$ can be determined as the RMS of the amplitude of a signal representation of the EEG signal for the selected segment/sample point.

The AR and MA coefficients for a selected segment are generated based on a time-invariant ARMA representation (i.e. based on Equation 2), so a signal representation of the EEG signal for that segment is generated based on Equation 2. The signal representation represents a sequence of values generated based on Equation 2 (i.e. $y[n]$ in Equation 2), where the output of Equation 2 is generated based on the estimated AR and MA coefficients for the selected segment, when driven by a normalised white noise input (i.e. where $u[n-k]$ represents random values determined by a zero mean unit variance Gaussian random process).

The AR and MA coefficients for a selected sample point are generated based on a time-varying ARMA representation (i.e. based on Equation 3). A signal representation of the EEG signal for that sample point is generated based on Equation 3. The signal representation represents a sequence of values (i.e. $y[n]$ in Equation 3), where the output of Equation 3 is generated based on the AR and MA coefficients for the selected sample point, when driven by a normalised white noise input (i.e. where $u[n-k]$ represents random values determined by a zero mean unit variance Gaussian random process).

ARMA gain can be generated in a number of ways, for example, using the arma2cor function of the ARMASA Matlab Toolbox application by P.M.T. Broersen of Delft University of Technology, or using any other ARMA modelling software package.

Equations 2 and 3 represent a fixed order (8,5) ARMA representation of a portion of an EEG signal. Although an ARMA representation having an autoregressive order of 8 and moving average order of 5 is expected to give the best results, other AR and MA orders can be selected.

Theoretically, the gain factor, g, depends on the parameters, q, according to Equation 19:

$$g \cong \frac{\exp(1)\psi_e[h_e^*(q)]\gamma_e}{\tau_e} \qquad \textbf{Equation 19}$$

and thus cortical input, $P(\omega)$, can be estimated using Equation 20:

$$P(\omega) \cong \frac{\tau_e \langle \widetilde{Y}(t) \rangle}{\exp(1)\psi_e[h_e^*(q)]\gamma_e \langle Y(t) \rangle} \qquad \textbf{Equation 20}$$

where, in Equations 19 and 20, $\psi_e[h_e^*(q)]$ represents the efficacy of excitation in cortex (and which is proportional to the transmembrane driving force for excitatory activity at rest), $\gamma_e$ represents the corresponding rate constant for excitation, and $\tau_e$ represents the effective passive membrane time constant. In mean field modelling, the respective values for $\psi_e[h_e^*(q)]$, $\gamma_e$ and $\tau_e$ during intervention are not expected to be significantly perturbed from their undisturbed values. The cortical input, $P(\omega)$, determined using Equation 20, together with the poles and zeros obtained from the coefficients $a_k$ and $b_k$ generated based on Equations 2 or 3, represents a more comprehensive linear characterisation of the dynamics of the EEG signal detected from the subject, than using the ARMA coefficients alone.

Thus, provided with knowledge about how a particular pharmaceutical agent affects single neuronal physiological properties, of which there is extensive information, the technique disclosed herein can be used to determine variations in input to the brain that are affected by said pharmaceutical agents. This is of particular relevance when it is considered that a variety of pharmaceutical agents known to affect brain function have sites and targets of action that are distributed throughout the central nervous system.

For example, nitrous oxide is both a hypnotic and analgesic agent and is known to affect sites cortically and subcortically (e.g. as discussed in Hopkins PM, Nitrous oxide: a unique drug of continuing importance for anaesthesia, Best Pract Res Clin Anaesthesiol., 2005, Sep, 19(3), pp.381-9; and Rudolph U & Antkowiak B., Molecular and neuronal substrates for general anaesthetics., Nat Rev Neurosci., 2004, Sep, 5(9), pp.709-20). Being able to non-invasively quantify both the levels of hypnosis and analgesia is of great clinical utility, as separate measures have important implications in terms of subsequent and ongoing clinical management and clinical outcome. For example, detecting adequate analgesia is important for achieving physiological (autonomic) stability during surgical procedures, and helps improve postoperative clinical outcomes. Quantifying the magnitude of subcortical input may provide one way of assessing the level of analgesia by monitoring the extent to which peripherally derived sensory information reaches cortex.

Attempts to monitor brain function for the purposes of assessing drug action or disease process necessarily depend upon determining what represents a physiologically meaningful measure. Because the human electroencephalogram (or EEG) has a time scale roughly commensurate with the time scale of cognition, in addition to it reflecting global aspects of cortical function, it has found favour in a wide range of studies in which an objective measure of drug action or disease process on higher brain function is required. However, the use of the EEG is compromised since the relationship between its various dynamical features and the underlying physiological mechanisms are in general ambiguous and poorly defined.

For instance, in Alzheimer's disease (AD) a number of EEG features have been identified that appear to correlate with cognitive impairment, and in addition to its potential diagnostic value, could by inference be used to assess partial or complete restitution of cognitive function in response to a range of novel pharmacological treatments or interventions. The hallmark of EEG abnormalities in AD is a slowing of the EEG's rhythms and a decrease in inter-regional/inter-areal coherence (as described for example in Brenner RP, Ulrich RF, Spiker DG, Sclabassi RJ, Reynolds III CF, Marin RS, Boller F. Computerized EEG spectral analysis in elderly normal, demented and depressed subjects, Electroencephalogr Clin Neurophysiol, 1986, 64, pp 483-92; Coben LA, Danziger W, Storandt M. A longitudinal EEG study of mild senile dementia of Alzheimer type. Electroencephalogr Clin Neurophysiol, 1985, 61, pp 101-12;; Giaquinto S, Nolfe G. The EEG in the normal elderly: a contribution to the interpretation of aging and dementia. Electroencephalogr Clin Neurophysiol, 1986, 63, pp 540-6; Dunkin JJ, Leuchter AF, Newton TF, Cook IA. Reduced EEG coherence in dementia: state or trait marker?, Biol Psychiatry, 1994, 35, pp 870-9; Locatelli T, Cursi M, Liberati D, Franceschi M, Comi G. EEG coherence in Alzheimer's disease. Electroencephalogr Clin Neurophysiol, 1998, 106, pp 229-37). Of particular relevance to the diagnostic use of EEG in AD is the fact that the detected EEG abnormalities appear to be correlated with the severity of the disease (see Brenner et al 1986; Kowalski et al 2001). Further, early empirical results suggest that EEG analysis may make possible the differentiation between AD and the subcortical/vascular dementias and a number of other senile and cognitive pathologies which include multisystem atrophy and depression.

It has been suggested that many of the EEG abnormalities seen in AD may be due to the degeneration of a number of ascending (afferent or input) pathways to cortex. For instance ascending cholinergic pathways (originating in the basal forebrain and upper pontine tegmentum), which diffusely innervate much of the cortex, are inferred to be impaired in AD, as demonstrated by the significant reduction of empirical markers of cholinergic neurotransmission. The partial success of drugs that increase cortical levels of acetylcholine (e.g. donepezil, tacrine, rivastigmine and galantamine) in improving memory and cognitive function in mild/moderate AD sufferers underscores the pathophysiological significance of acetylcholine. However degenerative changes in AD also affect a number of other ascending cortical input and modulatory pathways (see for example, and references contained within, Jeong J. EEG dynamics in patients with Alzheimer's disease. Clin Neurophysiol, 2004, 115, 1490-1505).

A further well established feature of AD (in addition to the slowing) is the decreased EEG coherence in the alpha (8-13 Hz) and beta (13-30 Hz) bands suggesting that changes in the connection between various cortical areas (through cortico-cortical fibres) may also represent a significant, clinically relevant, abnormality associated with AD. However, there are two main problems associated with coherence that complicate its use and interpretation. Firstly, there is the problem of choosing electrode pairs. For example, in principle, 64 recording electrodes requires that up to 2016 ($64 \times 63/2$) pair-wise coherences need to be evaluated. However it is not practical to analyse all pairs. Secondly, alterations in pair-wise coherence do not necessarily imply that function connectivity is altered as it may be input arising from a common source that has changed.

While EEG coherence and a range of QEEG methods (both linear (e.g. power spectral analysis) and non-linear (e.g. bicoherence, entropy, etc.)) are able to differentiate EEG from AD from the EEG of other CNS disorders, the analysis is not necessarily physiologically specific. This has implications not only in terms of diagnosis and monitoring the effects of drug treatment, but also in terms of the evaluation and design of new treatments. The embodiments of the present invention described herein may be used to overcome many of these restrictions. The value of new approaches to the analysis of EEG has been emphasised by Jeong (2004) which recognises that novel methods for EEG analysis will help improve the accuracy of early detection of AD.

In one embodiment of the present invention, the system 100 generates cortical state data and cortical input data for each EEG segment/sample point respectively. Cortical state data for a subject is generated based on the value of the first AR coefficient (i.e. the coefficient $a_1$) for the corresponding EEG segment/sample point, and cortical input data for a subject is generated based on Equation 18. The system 100 generates, based on the cortical state data and cortical input data, a time-based graphical representation for representing the changes in the correlation between a subject's cortical state and level of cortical input at different points in time.

Figure 10 shows an example of a graphical representation 1000 of a CI-CS plane generated by the system 100. The vertical axis 1002 of the graphical representation 1000 represents the relative cortical state (CS), and the horizontal axis 1004 represents the relative cortical input (CI). The correlation of the values represented by the cortical state data and cortical input data for each respective EEG segment/sample is represented as a point on the graphical representation 1000. Thus, for a sequence of EEG segments/samples, the graphical representation 1000 is populated with a number of points at various positions, which represents the changes in cortical state and cortical input based on time.

Referring to the example shown in Figure 10, a first point 1006 represents the correlation of cortical state/input values for the first EEG segment/sample (i.e. at t=0, where *t* represents time). A second point 1008 represents the correlation of cortical state/input values at the time when the subject losses consciousness. A third point 1009 represents the correlation of cortical state/input values for a final EEG segment/sample. In one embodiment, the points on the graphical representation 1000 for adjacent EEG segments/samples are joined together by a line, so that the graphical representation 1000 represents a sequence of EEG segments/samples as a line segment that evolves or varies relative to time. The values represented by the cortical state data and/or cortical input data may be either absolute values, or relative values generated relative to a reference point on the representation 1000. For example, the reference point may represent the cortical state/input values at a particular point in time, or when the subject is at a particular state of consciousness (e.g. awake or unconscious), based on the average position of groups of points (or clusters) on the graphical representation 1000.

Different states of the brain are represented by points at different regions of the graphical representation 1000 of the CI-CS plane. For example, as shown in Figure 10, the graphical representation 1000 may be divided into two regions 1010 and 1012 by a border line 1014, where region 1010 represents a state of unconsciousness and region 1012 represents a state of alertness (e.g. the subject being awake). If the correlation of cortical state/input values generates a point that falls within a first region 1012, then the subject would be classified awake at the time corresponding to that point. Similarly, if the correlation of cortical state/input values generates a point that falls within a second region 1010, then the subject would be classified unconscious at the time corresponding to that point. The border line 1014 may be generated for display as part of the graphical representation 1000.

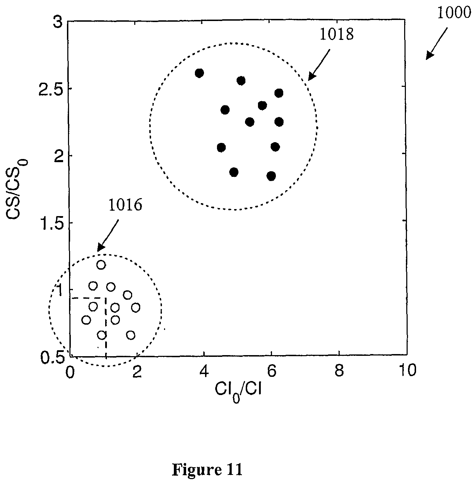

Many different regions (e.g. of different shapes and positions) can be identified within the graphical representation 1000. For example, as shown in Figure 11, the cluster of points within control region 1016 may represent a subject being in a state of rest. Alternatively, the cluster of points within control region 1016 may be specifically selected based on other criteria to serve as a control, for example, to represent the correlation of cortical state/input values at a particular point in time, or at the outset of an EEG recording. The other points on the graphical representation 1000 are generated with reference to the points in the control region 1016. The points in region 1018 may represent a state of brain function corresponding to an abnormal (e.g. due to some degenerative disease process such as Alzheimer's disease, Parkinson's disease or any other neurodegenerative process) or induced state (e.g. due to the effects of one or more pharmaceutical/nutriceutical or other drug agents).

If multiple sufficiently closely spaced electrodes have been used to record EEG, as for example with the international 10-20 system of EEG electrode placement, then interpolated spatial maps may be created indicating the value of CI and CS at a given scalp location at a give time, in addition to the CI-CS plane plots for each electrode discussed above. EEG recorded from nearby, closely separated, electrodes will inevitably contain correlations due to the effects of volume conduction, which will reduce the spatial resolution of any subsequently generated spatial maps of CS and CI. Therefore, if multiple recording electrodes are used, a method that ameliorates the effects of this volume conduction could be used (as for example discussed in Ferree TC. Spherical splines and average referencing in scalp electroencephalography. Brain Topogr, 2006, Oct 4; Srinivasan R, Nunez PL, Silberstein R. Spatial filtering and neocortical dynamics: estimates of EEG coherence, IEEE Trans Biomed Eng, 1998, 45, pp 814-26; Freeman WJ. Use of spatial deconvolution to compensate for distortion of EEG by volume conduction. IEEE Trans Biomed Eng, 1980, 27, pp 421-9) before any subsequent ARMA analysis is performed.

The interpolated maps referred to above can be created using a variety of approaches (as summarised in Soong AC, Lind JC, Shaw GR, Koles ZJ. Systematic comparisons of interpolation techniques in topographic brain maping. Electroencephalogr Clin Neurophysiol, 1993, 87, pp 185-95), and in general are generated over a three-dimensional head model, that may be generic, but which is more typically constructed from the subjects electrode locations. The maps generated using this approach are generally colored or shaded according to the magnitude of the interpolated variable at a given scalp spatial location.

Because different regions of the CI-CS plane are predicted to correspond to different brain states such a display offers the possibility of comparing and characterizing different clinical groups and of developing appropriate heuristic/statistical discriminating functions between these groups. Such heuristic discriminating functions may be evaluated from EEG recorded simultaneously from one or a number of EEG electrodes. For N electrodes and two derived measures per electrode, CS and CI, a total of 2N variables can be used as the basis for determining a discriminant function to statistically separate two or more patient/subject population grouped according to some clinical or non-clinical variable or criteria. Such a discriminate function may be calculated using any of a variety of statistical software packages such as SPSS (SPSS Inc, Chicago, IL, USA).

An advantage provided by the embodiments described herein in comparison with other processed EEG approaches is that changes in cortical function (e.g. due to AD induced damage of cortical tissue) can be separated from changes in cortical input (e.g. due to AD induced damage of subcortical input/modulatory pathways). This distinction is particularly useful in characterising the effects of anaesthetic agents which separately modify hypnotic state (cortical function) and pain responsiveness (cortical input). Thus, the principles related to the preferred embodiments have relevance in assessing how Alzheimer's disease (as well as any other disease process that affects higher brain function) differentially affects brain function. This will help guide the search for optimal drug therapies by their effectiveness, for example, by enabling the determination of which aspects of higher brain function should be targeted for optimal treatment or diagnostic efficacy.

The preferred embodiments of the present invention may be used to monitor abnormalities affecting higher brain function arising from various conditions, disorders or diseases, including for example:

- Alzheimer's disease;
- Vascular dementias (e.g. mild vascular cognitive impairment, multi-infarct dementia, Biswanger's disease, mixed dementia, single infarct vascular dementia);
- Subcortical dementias (e.g. Huntingtons, Multiple Sclerosis, Parkinson's disease);
- Pick's (frontotemporal dementia) disease and other frontal lobe dementias;
- Depression and other psychiatric pathologies; and/or
- Other diseases/conditions impairing cognition and brain function (such as drug induced encephalopathy, and multi-system atrophy).

The preferred embodiments can also be used on a subject by subject basis for diagnosis to determine the presence or absence of one or more particular conditions, disorders or diseases (such as those listed above), or alternatively, used to obtain readings before, after or during administration of a particular treatment (e.g. including drugs or a course of physical/mental activity) to determine the effectiveness of that treatment.

It will be appreciated that the process of the invention is not, in itself, in any way concerned with therapeutic treatment of the patient. Rather, it is concerned with monitoring the conditions, disorders or diseases such as those referred to above without causing any change in the subject or attempting to make any change in the subject. In cases where there is pharmacological intervention, the process of the invention again does not have any direct affect in the efficacy of the intervention but rather is used to determine the effectiveness of the intervention.

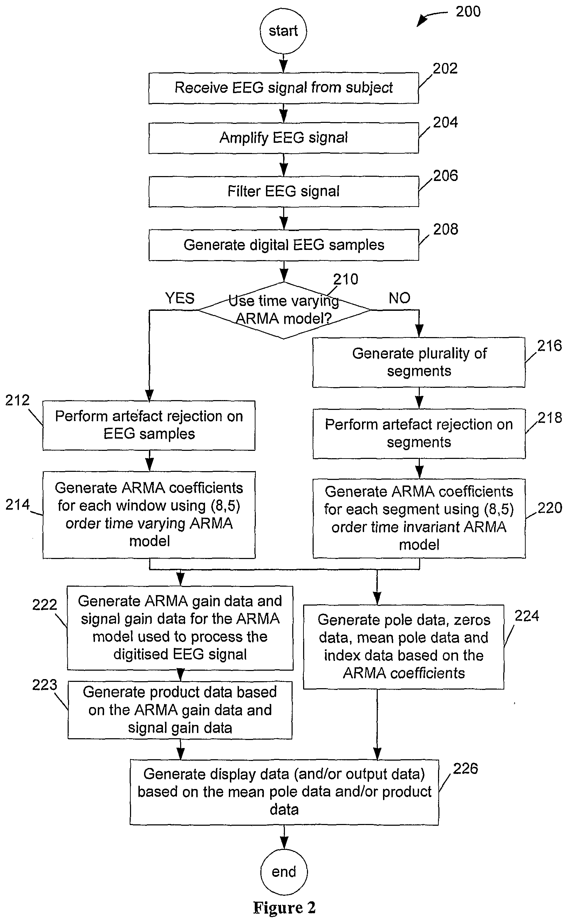

Figure 2 is a flow diagram of an EEG analysis process 200 performed by the EEG processing system 100. Process 200 begins at step 202 with the signal processing module 106 receiving an EEG signal from the subject via the electrodes 102. At step 204, module 106 amplifies the EEG signal. At step 208, module 106 converts the EEG signal into digital EEG samples. At step 206, module 106 filters the EEG signal using a band-pass filter to remove low frequency movement artefacts, EMG components and main artefacts (generally arising between 0 Hz to 50-60 Hz), as well as other sources of external noise.

Step 210 decides how to process the EEG samples based on the user's selection. If the user selects a processing option for analysing the samples substantially in real-time by recursively estimating the AR and MA coefficients (Equations 6 and 7), step 210 proceeds to step 212. Otherwise, a default processing option is selected to estimate the AR and MA coefficients (Equation 2) and step 210 proceeds to step 216.

The response index calculation module 108 performs steps 212 and 214 in respect of each new sample point generated by the module 108. At step 212, module 108 performs additional artefact rejection on the EEG samples, including further removal of periodic 0 Hz to 50-60 Hz interference from the samples, which is known to compromise the subsequent estimation of AR and MA coefficients.

At step 214, module 108 generates coefficient data for each sample point representing the AR and MA coefficients for a time varying ARMA representation of the EEG signal for each sampled EEG value obtained by digitisation. For example, at step 214, module 108 generates the AR and MA coefficients directly from the EEG samples using a Kalman adaptive filtering method. In step 214, the coefficient data represents the optimal values of the AR and MA coefficients as described above. Step 214 then proceeds to step 222 and step 224, which are performed in parallel to each other. The time varying coefficient data can be generated using software, such as the functions associated with the MATLAB® System Identification Toolbox version 6.0). Step 220 then proceeds to step 222 and step 224.

At step 216, module 108 generates a plurality of segments, each representing a portion of the digital EEG signal for the same duration. For example, each segment may represent a 2-second sample of the EEG signal and may overlap with a portion of an adjacent segment. At step 218, module 108 filters each segment to remove additional artefacts from the signal, including removing periodic 0 Hz to 50-60 Hz interference which is known to compromise the subsequent estimation of AR and MA coefficients. At step 220, module 108 generates coefficient data based on the respective time invariant ARMA representation of the EEG signal (e.g. based on an (8,5) order ARMA representation) for each segment. Estimates of the AR and MA coefficients for each fixed length segment can be generated in a number of ways, for example, using the ARMASA Matlab Toolbox application by P.M.T. Broersen of Delft University of Technology, or using any other ARMA modelling software package.

At step 222, module 108 generates, for each segment/sample point, ARMA gain data representing an ARMA gain value (i.e. $\langle Y(t) \rangle$ in Equation 18) based on the corresponding ARMA representation used in step 214 or 220 for generating the coefficient data for the corresponding segment/sample point. This involves applying the estimated AR and MA coefficients to the corresponding ARMA representation (i.e. Equation 2 or Equation 10), and generating a signal representation (represented by $y[n]$) by driving the corresponding ARMA representation with a normalised white noise input (represented by $u[n-k]$).

At step 222, module 108 also generates, for each segment/sample point, signal gain data representing a signal gain value (i.e. $\langle \tilde{Y}(t) \rangle$ in Equation 18) based on the EEG samples for the corresponding segment, in the case where a time-invariant ARMA representation is used (at steps 216, 218, and 220), or for a range of EEG sample values in the case where a time varying ARMA representation is used (at steps 212 and 214). The value of $\langle \tilde{Y}(t) \rangle$ for each sample

point is generated based on an appropriate portion of the detected EEG signal either centred on the time at which the coefficient data for the corresponding sample point was generated, or generated based on an appropriately constructed average of the sampled EEG signal including and prior to the current sample point. Alternatively, a value of $\langle \tilde{Y}(t) \rangle$ is generated based on each respective segment generated by module 108 at step 216.

At step 223, module 108 then generates, for each segment/sample point, cortical input data representing the value of the product $g(q)P(\omega)$ according to Equation 18 based on the corresponding ARMA gain data and signal gain data. Changes in the value of the $g(q)P(\omega)$ represents changes in the magnitude of the subcortical input (represented by $P(\omega)$).

Step 224, generates poles data and zeros data based on Equations 9 and 10, and then module 108 generates mean pole data based on Equation 12 representing the unsealed mean pole position for each respective segment/sample point. Module 108 then generates index data based on Equations 13 or 14 representing a scaled numeric representation of the mean pole position for the corresponding segment/sample point.

Steps 224 and 223 proceed to step 226 for display. At step 226, the display module 114 generates display data representing user interfaces based on brain state (e.g. using the poles data, zeros data, mean pole data, index data) and brain (cortical) input data, to represent the functional state of the subject's brain and/or level of subcortical activity in the subject's brain. The display data generated at step 226 includes a graphical representation (generated based on the cortical input data and cortical state data for different EEG segments/samples) of the correlation between cortical state values and cortical input values for different EEG segments/samples over a predefined period of time (e.g. for the duration of an EEG recording). Step 226 also controls the data export module to generate output data including data representative of the EEG samples, brain state data, raw mean pole data and/or scaled mean pole data. Process 200 ends after step 226.

Changes in the value represented by the scaled mean pole data (or unsealed mean pole data) represent changes in the functional state of the subject's brain (i.e. how the brain responds to cortical input). Changes in the value represented by cortical input data represent changes in value of the product $g(q)P(\omega)$ and thus the level of brain cortical input. An advantage provided by the ARMA gain, and hence a measure of subcortical input, is to enhance the physiological specificity (and hence clinical utility) of the determination of the subject's brain function.

Many modifications will be apparent to those skilled in the art without departing from the scope of the present invention as herein described with reference to the accompanying drawings.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

**Claims**

1. A method for analysing an electroencephalogram representative of the functional state of the brain of a subject, the brain having a cortex and being subject to cortical and subcortical inputs, the method including the steps of:

    (i) obtaining electroencephalogram (EEG) signals from a plurality of electrodes positioned on the subject's scalp;
    (ii) generating a plurality of EEG segments from the EEG signals;
    (iii) representing said EEG segments as a fixed-order autoregressive moving average (ARMA) signal representation with an autoregressive order (AR) between 8 and 14 and a moving average (MA) order between 5 and 11;
    (iv) generating AR and MA coefficient data of said ARMA signal representation for said EEG segments;
    (v) generating, based on said AR and MA coefficient data, cortical state (CS) data representing the functional state of said cortex at times corresponding to respective EEG segments;
    (vi) generating cortical input data (CI) from said AR and MA coefficient data and said EEG segments representative of the level of cortical and subcortical inputs to said cortex at times corresponding to respective EEG segments;
    (vii) generating, based on said cortical state (CS) data and said cortical input (CI) data generated in steps (v) and (vi), display data in the form of a sequence of CS and CI pairs at the same points in time as the corresponding EEG segments; and
    (viii) displaying said display data on display means.

2. A method as claimed in claim 1 including the step of:
    (ix) providing the user of the method an option to select a time varying or time invariant ARMA signal representation in step (iv).

3. A method as claimed in claim 2 including the steps of:

(x) selecting a fixed order time invariant ARMA signal representation of autoregressive order 8 and moving average order of 5 to describe each of the EEG segments by a first equation;

$$y[n] = -\sum_{k=1}^{8} a_k y[n-k] + \sum_{k=0}^{5} b_k u[n-k]$$

where $y[n]$ represents an ordinal sequence of samples of said EEG signal of said segment, $y[n-k]$ represents the $k$-th prior sampled value of $y[n]$; $u[n-k]$ represents a Gaussian white noise process; and $a_k$ and $b_k$ represent the estimated autoregressive AR and moving average MA coefficients generated in step (iv) respectively for a said segment of said EEG signal;

(xi) solving the first equation to determine AR coefficients $a_1$ - $a_8$ and MA coefficients $b_0$ - $b_5$;

(xii) performing a z-transform on the first equation to obtain a z-domain representation given by a second equation;

$$Y(z) = \frac{\sum_{k=0}^{5} b_k z^{-k}}{\sum_{k=0}^{8} a_k z^{-k}} U(z)$$

where Y(z) represents an ARMA representation of each portion of the EEG signal in the z-domain; and U(z) represents a Gaussian white noise process in the z-domain;

(xiii) substituting each of the values of the coefficients obtained in step (xi) into the second equation of step (xii);

(xiv) determining poles of the second equation of step (xii) using the equation;

$$\sum_{k=0}^{8} a_k p^{-k} = \sum_{k=0}^{8} a_k p^{8-k} = 0$$

wherein there are 8 poles each represented by the symbol $p$; and

(xv) calculating the sum of the poles determined in step (xiv) using either;

(a)

$$\overline{z}_p \equiv \sum_{i=1}^{i=8} z_{i,p} \quad ;$$

or

(b) $\overline{z}_p = a_1$ wherein $a_1$ is the first autoregressive coefficient of said time invariant ARMA signal representation and wherein $z_{i,p}$ represents the i-th pole.

(xvi) determining the mean pole ($\overline{z}_p/8$) of said EEG segments as the sum of the poles, calculated in step (xv), by dividing by 8;

(xvii) substituting the AR and MA coefficients determined in step (xi) into the first equation in step (x) and calculating amplitude values of the said EEG segments for a normalized white noise input;

(xviii) generating an ARMA gain value $\langle Y(t) \rangle$ as the root mean square value of the amplitude values for said signal representations determined in step (xvii);

(xix) estimating the input to cortex as the product $g(q)P(\omega)$

$$g(q)P(\omega) = \frac{\langle \tilde{Y}(t) \rangle}{\langle Y(t) \rangle}$$

where $\langle Y(t) \rangle$ is the ARMA gain determined in step (xvii) above and $\langle \tilde{Y}(t) \rangle$ is the root mean squared of the amplitude of said EEG segments;

(xx) calculating the measure Cortical Input (CI) as the input to cortex generated in step (xix); and

(xxi) calculating the measure Cortical State (CS) as the mean pole generated in step (xvi).

4. A method as claimed in claim 2 including the steps of:

(xxii) selecting a fixed order time varying ARMA signal representation of autoregressive order 8 and moving average order of 5 to describe each of the EEG segments by a first equation;

$$y[n] = -\sum_{k=1}^{8} a_k^{(n)} y[n-k] + \sum_{k=0}^{5} b_k^{(n)} u[n-k]$$

wherein $a_k^{(n)}$ and $b_k^{(n)}$ represent the estimated autoregressive and moving average coefficients generated in step (iv) respectively for a said segment of said EEG signal for time instant n;

(xxiii) recursively estimating the time varying autoregressive coefficients $a_1^{(n)} - a_8^{(n)}$ and time varying moving average coefficients $b_0^{(n)} - b_5^{(n)}$ for time instant $n$;

(xxiv) performing a z-transform on said first equation to obtain a z-domain representation given by a second equation;

$$Y(z) = \frac{\sum_{k=0}^{5} b_k z^{-k}}{\sum_{k=0}^{8} a_k z^{-k}} U(z)$$

where $Y(z)$ represents an ARMA representation of a portion of the EEG signal in the z-domain and $U(z)$ represents a Gaussian white noise process in the z-domain;

(xxv) determining the 8 poles for time instant $n$ as solutions to the said second equation;

(xxvi) determining the sum $(\bar{z}_p)$ of said poles for time instant $n$ as either;

(a)

$$\bar{z}_p \equiv \sum_{i=1}^{i=8} z_{i,p} \quad ;$$

or

(b) $\bar{z}_p = a_1^{(n)}$ wherein $a_1^{(n)}$ is the first autoregressive coefficient of said time varying ARMA signal representation of autoregressive order 8 and moving average order of 5 at time instant $n$;

(xxvii) determining the mean pole $(\bar{z}_p/8)$ of said segments of said EEG signals as the sum of poles, calculated in step (xxvi), by dividing by 8;

(xxviii) substituting the AR and MA coefficients determined in step (xxiii) for each time instant $n$ into the first equation of step (xxii) and calculating amplitude values of the said EEG segments for a normalized white noise input;

(xxix) generating an ARMA gain value $\langle Y(t)\rangle$ for each time step $n$ as the root mean square value of the amplitude values for said signal representation determined in step (xxviii) for said time step $n$;

(xxx) estimating the input to cortex as the product $g(q)P(\omega)$ calculated using the equation;

$$g(q)P(\omega) = \frac{\langle \tilde{Y}(t)\rangle}{\langle Y(t)\rangle}$$

where $\langle Y(t)\rangle$ is the ARMA gain determined in step (xxix) and where $\langle \tilde{Y}(t)\rangle$ represents the average signal amplitude of a portion of the EEG signal estimated for each time instant $n$ as the root mean square of the EEG signal obtained by using a fixed length sliding window;

(xxxi) calculating the measure Cortical Input (CI) as the input to cortex generated in step (xxx); and

(xxxii) calculating the measure Cortical State (CS) as the mean pole generated in step (xxvii).

5. A method as claimed in claim 3 or 4 wherein the Cortical State (CS) is scaled to fall within a predefined range.

6. A method as claimed in claim 3, 4 or 5 wherein the Cortical Input (CI) is scaled to fall within a predefined range.

7. A method as claimed in any of claims 1 to 6 wherein the generated values of said CS and CI pairs are graphically represented as points in a plane, the CS-CI plane, having a horizontal axis defined over a range of CS values and a vertical axis defined over a range of CI values.

8. A method as claimed in claim 7 whereby a heuristic/statistical discriminating function is constructed to define regions of the CS-CI plane having differing functional properties with respect to brain activity.

9. A method as claimed in any one of claims 1 to 6 wherein the generated values of said CS and CI pairs are separately graphically represented as an interpolated coloured or shaded topographic map, the spatial mapping of which is calculated to depend on the physical locations of the respective locations of the electrodes on the subject's scalp.

10. A system for analysing an electroencephalogram representative of the functional state of a brain of a subject, the brain having a cortex and being subject to cortical and subcortical inputs, the system including:

a plurality of electrodes (102) for positioning on the subject's scalp for generating electroencephalogram (EEG) signals;

a processor module (108); and

display means (104),

said processor module (108) being coupled to receive the (EEG) signals from the electrodes and adapted to:

(i) generate a plurality of EEG segments from the EEG signals;

(ii) represent said EEG segments as a fixed-order autoregressive moving average (ARMA) signal representation with an autoregressive order (AR) between 8 and 14 and a moving average (MA) order between 5 and 11;

(iii) generate AR and MA coefficient data of said ARMA signal representation for said EEG segments;

(iv) generate, based on said AR and MA coefficient data, cortical state (CS) data representing the functional state of said cortex at times corresponding to respective EEG segments;

(v) generate cortical input (CI) data from said AR and MA coefficient data and said EEG segments representative of the level of subcortical and subcortical inputs to said cortex at times corresponding to respective EEG segments;

(vi) generate, based on said cortical state (CS) data and said cortical input (CI) data generated in steps (iv) and (v), display data in the form of a sequence of CS and CI pairs at the same points in time as the corresponding EEG segments ; and wherein the display means (104) is operable to display the display data.

11. A system as claimed in claim 10 wherein the processor module (108) is operable to:

(vii) provide the user of the method an option to select a time varying or time invariant ARMA signal representation in step (iii).

**12.** A system as claimed in claim 11 wherein the processor module (108) is operable to:

(viii) select a fixed order time invariant ARMA signal representation of autoregressive order 8 and moving average order of 5 to describe each of the EEG segments by a first equation;

$$y[n] = -\sum_{k=1}^{8} a_k y[n-k] + \sum_{k=0}^{5} b_k u[n-k]$$

where $y[n]$ represents an ordinal sequence of samples of said EEG signal of said segment, $y[n-k]$ represents the $k$-th prior sampled value of $y[n]$; $u[n-k]$ represents a Gaussian white noise process; and $a_k$ and $b_k$ represent the estimated autoregressive AR and moving average MA coefficients generated in step (iii) respectively for a said segment of said EEG signal;

(ix) solve the first equation to determine AR coefficients $a_1$ - $a_8$ and MA coefficients $b_0$ - $b_5$;

(x) perform a z-transform on the first equation to obtain a z-domain representation given by a second equation;

$$Y(z) = \frac{\sum_{k=0}^{5} b_k z^{-k}}{\sum_{k=0}^{8} a_k z^{-k}} U(z)$$

(xi) substitute each of the values of the coefficients obtained in step (ix) into the second equation of step (x);

(xii) determine poles of the second equation of step (x) using the equation;

$$\sum_{k=0}^{8} a_k p^{-k} = \sum_{k=0}^{8} a_k p^{8-k} = 0$$

wherein there are 8 poles each represented by the symbol $p$; and

(xiii) calculate the sum of the poles determined in step (xii) using either;

(a)

$$\overline{z}_p \equiv \sum_{i=1}^{i=8} z_{i,p} \quad ;$$

or

(b) $\overline{z}_p = a_1$ wherein $a_1$ is the first autoregressive coefficient of said time invariant ARMA signal representation.

(xiv) determine the mean pole ($\overline{z}_p/8$) of said EEG segments as the sum of the poles, calculated in step (xiii), by dividing by 8;

(xv) substitute the AR and MA coefficients determined in step (ix) into the first equation in step (viii) and calculating amplitude values of the said EEG segments for a normalized white noise input;

(xvi) generate an ARMA gain value $\langle Y(t) \rangle$ as the root mean square value of the amplitude values for said signal representations determined in step (xv);

(xvii) estimate the input to cortex as the product $g(q)P(\omega)$

$$g(q)P(\omega) = \frac{\langle \tilde{Y}(t) \rangle}{\langle Y(t) \rangle}$$

where $\langle Y(t) \rangle$ is the ARMA gain determined in step (xvii) above and $\langle \tilde{Y}(t) \rangle$ is the root mean squared of the amplitude of said EEG segments;

(xviii) calculate the measure Cortical Input (CI) as the input to cortex generated in step (xvii); and

(xix) calculate the measure Cortical State (CS) as the mean pole generated in step (xiv).

**13.** A system as claimed in claim 11 wherein the processor module is operable to:

(xx) select a fixed order time varying ARMA signal representation of autoregressive order 8 and moving average order of 5 to describe each of the EEG segments by a first equation;

$$ y[n] = -\sum_{k=1}^{8} a_k^{(n)} y[n-k] + \sum_{k=0}^{5} b_k^{(n)} u[n-k] $$

wherein $a_k^{(n)}$ and $b_k^{(n)}$ represent the estimated autoregressive and moving average coefficients generated in step (iii) respectively for a said segment of said EEG signal for time instant n;

(xxi) recursively estimate the time varying autoregressive coefficients $a_1^{(n)} - a_8^{(n)}$ and time varying moving average coefficients $b_0^{(n)} - b_5^{(n)}$ for time instant $n$;

(xxii) perform a z-transform on said first equation to obtain a z-domain representation given by a second equation;

$$ Y(z) = \frac{\sum_{k=0}^{5} b_k z^{-k}}{\sum_{k=0}^{8} a_k z^{-k}} U(z) $$

where $Y(z)$ represents an ARMA representation of a portion of the EEG signal in the z-domain and $U(z)$ represents a Gaussian white noise process in the z-domain;

(xxiii) determine the 8 poles for time instant $n$ as solutions to the said second equation;

(xxiv) determine the sum $(\bar{z}_p)$ of said poles for time instant $n$ as either;

(a)

$$ \bar{z}_p \equiv \sum_{i=1}^{i=8} z_{i,p} \quad ; $$

or

(b) $\bar{z}_p = a_1^{(n)}$ wherein $a_1^{(n)}$ is the first autoregressive coefficient of said time varying ARMA signal representation of autoregressive order 8 and moving average order of 5 at time instant $n$;

(xxv) determine the mean pole $(\bar{z}_p/8)$ of said segments of said EEG signals as the sum of poles, calculated in step (xxiv), by dividing by 8;

(xxvi) substitute the AR and MA coefficients determined in step (xxi) for each time instant $n$ into the first equation of step (xx) and calculating amplitude values of the said EEG segments for a normalized white noise input;

(xxvii) generate an ARMA gain value $\langle Y(t) \rangle$ for each time step $n$ as the root mean square value of the amplitude values for said signal representation determined in step (xxvi) for said time step $n$;

(xxviii) estimate the input to cortex as the product $g(q)P(\omega)$ calculated using the equation;

$$g(q)P(\omega) = \frac{\langle \tilde{Y}(t) \rangle}{\langle Y(t) \rangle}$$

where $\langle Y(t) \rangle$ is the ARMA gain determined in step (xxvii) and where $\langle \tilde{Y}(t) \rangle$ represents the average signal amplitude of a portion of the EEG signal estimated for each time instant $n$ as the root mean square of the EEG signal obtained by using a fixed length sliding window;

(xxix) calculate the measure Cortical Input (CI) as the input to cortex generated in step (xxviii); and

(xxx) calculate the measure Cortical State (CS) as the mean pole generated in step (xxv).

14. Computer executable code stored on a computer readable medium which, when executed by the processor module of the system as defined in any one of claims 10 to 13, causes the processor module to perform the steps in the method as defined in any one of claims 1 to 9.

**Patentansprüche**

1. Verfahren zur Analyse eines Elektroenzephalogramms, das für den Funktionszustand des Gehirns eines Subjekts repräsentativ ist, wobei das Gehirn einen Kortex hat und kortikalen und subkortikalen Eingängen unterliegt, wobei das Verfahren die folgenden Schritte beinhaltet:

(i) Gewinnen von EEG-(Elektroenzephalogramm)-Signalen von mehreren Elektroden, die auf der Kopfhaut des Subjekts positioniert sind;

(ii) Erzeugen mehrerer EEG-Segmente anhand der EEG-Signale;

(iii) Darstellen der genannten EEG-Segmente als eine ARMA-(Autoregressive Moving Average)-Signaldarstellung fester Ordnung mit einer autoregressiven Ordnung (AR) zwischen 8 und 14 und einer MA-(Moving Average)-Ordnung zwischen 5 und 11;

(iv) Erzeugen von AR- und MA-Koeffizientendaten der genannten ARMA-Signaldarstellung für die genannten EEG-Segmente;

(v) Erzeugen, anhand der genannten AR- und MA-Koeffizientendaten, von Kortexzustands-(CS)-Daten, die den Funktionszustand des genannten Kortex zu Zeiten darstellen, die den jeweiligen EEG-Segmenten entsprechen;

(vi) Erzeugen von Kortexeingangsdaten (CI) anhand der genannten AR- und MA-Koeffizientendaten und der genannten EEG-Segmente, die den Level an kortikalen und subkortikalen Eingängen in den genannten Kortex zu Zeiten repräsentieren, die den jeweiligen EEG-Segmenten entsprechen;

(vii) Erzeugen, anhand der in den Schritten (v) und (vi) erzeugten genannten Kortexzustands-(CS)-Daten und genannten Kortexeingangs-(CI)-Daten, von Anzeigedaten in Form einer Sequenz von CS- und CI-Paaren zu den gleichen Zeitpunkten wie die entsprechenden EEG-Segmente; und

(viii) Anzeigen der genannten Anzeigedaten auf Anzeigemitteln.

2. Verfahren nach Anspruch 1, das den folgenden Schritt beinhaltet:
(ix) Geben, dem Anwender des Verfahrens, einer Option zur Auswahl einer zeitvarianten oder zeitinvarianten ARMA-Signaldarstellung in Schritt (iv).

3. Verfahren nach Anspruch 2, das die folgenden Schritte beinhaltet:

(x) Auswählen einer zeitinvarianten ARMA-Signaldarstellung fester Ordnung mit der autoregressiven Ordnung 8 und der MA-Ordnung 5, um jedes der EEG-Segmente durch eine erste Gleichung zu beschreiben:

$$y[n] = -\sum_{k=1}^{8} a_k y[n-k] + \sum_{k=0}^{5} b_k u[n-k]$$

wobei *y[n]* eine ordinale Sequenz von Samples des genannten EEG-Signals des genannten Segments repräsentiert, *y[n-k]* den *k*-ten vorher abgetasteten Wert von *y[n]* repräsentiert; *u[n-k]* einen Gaußschen Weißrausch-

prozess repräsentiert; und $a_k$ und $b_k$ die geschätzten autoregressiven AR- und MA-(Moving Average)-Koeffizienten repräsentieren, die in Schritt (iv) jeweils für ein genanntes Segment des genannten EEG-Signals erzeugt wurden;

(xi) Lösen der ersten Gleichung zur Bestimmung der AR-Koeffizienten $a_1$ - $a_8$ und der MA-Koeffizienten $b_0$ - $b_5$,

(xii) Durchführen einer z-Transformation an der ersten Gleichung, um eine z-Domänendarstellung zu erhalten, die durch eine zweite Gleichung angegeben wird:

$$Y(z) = \frac{\sum_{k=0}^{5} b_k z^{-k}}{\sum_{k=0}^{8} a_k z^{-k}} U(z)$$

wobei Y(z) eine ARMA-Darstellung jedes Teils des EEG-Signals in der z-Domäne repräsentiert; und U(z) einen Gaußschen Weißrauschprozess in der z-Domäne repräsentiert;

(xiii) Substituieren jedes der Werte der in Schritt (xi) erhaltenen Koeffizienten in die zweite Gleichung von Schritt (xii);

(xiv) Bestimmen von Polen der zweiten Gleichung von Schritt (xii) mittels der folgenden Gleichung:

$$\sum_{k=0}^{8} a_k p^{-k} = \sum_{k=0}^{8} a_k p^{8-k} = 0$$

wobei es 8 Pole gibt, jeweils durch das Symbol $p$ repräsentiert; und

(xv) Berechnen der Summe der in Schritt (xiv) bestimmten Pole mittels

    (a)

$$\bar{z}_p = \sum_{i=1}^{i=8} z_{i,p},$$

oder

(b) $\bar{z}_p = a_1$, wobei $a_1$ der erste autoregressive Koeffizient der genannten zeitinvarianten ARMA-Signaldarstellung ist und wobei $z_{i,p}$ den i-ten Pol repräsentiert;

(xvi) Bestimmen des mittleren Pols ($\bar{z}_p/8$) der genannten EEG-Segmente als Summe der Pole, berechnet in Schritt (xv), durch Dividieren durch 8;

(xvii) Substituieren der in Schritt (xi) bestimmten AR- und MA-Koeffizienten in die erste Gleichung in Schritt (x) und Berechnen von Amplitudenwerten der genannten EEG-Segmente für einen normalisierten Weißrauscheingang;

(xviii) Erzeugen eines ARMA-Verstärkungswertes $\langle Y(t) \rangle$ als quadratischen Mittelwert der Amplitudenwerte für die genannten Signaldarstellungen wie in Schritt (xvii) bestimmt;

(xix) Schätzen des Eingangs zum Kortex als das Produkt $g(q)P(\omega)$

$$g(q)P(\omega) = \frac{\langle \tilde{Y}(t) \rangle}{\langle Y(t) \rangle}$$

wobei ⟨Y(t)⟩ die in Schritt (xvii) oben bestimmte ARMA-Verstärkung ist und ⟨Ỹ(t)⟩ der quadratische Mittelwert der Amplitude der genannten EEG-Segmente ist;

(xx) Berechnen des Kortexeingangs-(CI)-Maßes als den in Schritt (xix) erzeugten Eingang zum Kortex; und

(xxi) Berechnen des Kortexzustands-(CS)-Maßes als den in Schritt (xvi) erzeugten mittleren Pol.

4. Verfahren nach Anspruch 2, das die folgenden Schritte beinhaltet:

(xxii) Auswählen einer zeitvarianten ARMA-Signaldarstellung fester Ordnung mit der autoregressiven Ordnung 8 und der MA-Ordnung 5, um jedes der EEG-Segmente durch eine erste Gleichung zu beschreiben:

$$y[n] = -\sum_{k=1}^{8} a_k^{(n)} y[n-k] + \sum_{k=0}^{5} b_k^{(n)} u[n-k]$$

wobei $a_k^{(n)}$ und $b_k^{(n)}$ die geschätzten autoregressiven und MA-Koeffizienten repräsentieren, die in Schritt (iv) jeweils für ein genanntes Segment des genannten EEG-Signals für Zeitpunkt $n$ erzeugt wurden;

(xxiii) rekursives Schätzen der zeitvarianten autoregressiven Koeffizienten $a_1^{(n)}$ - $a_8^{(n)}$ und der zeitvarianten MA-Koeffizienten $b_0^{(n)} - b_5^{(n)}$ für Zeitpunkt $n$;

(xxiv) Durchführen einer z-Transformation an der genannten ersten Gleichung, um eine z-Domänendarstellung zu erhalten, die durch eine zweite Gleichung angegeben wird:

$$Y(z) = \frac{\sum_{k=0}^{5} b_k z^{-k}}{\sum_{k=0}^{8} a_k z^{-k}} U(z)$$

wobei Y(z) eine ARMA-Darstellung eines Teils des EEG-Signals in der z-Domäne repräsentiert und U(z) einen Gaußschen Weißrauschprozess in der z-Domäne repräsentiert;

(xxv) Bestimmen der 8 Pole für den Zeitpunkt $n$ als Lösungen für die genannte zweite Gleichung;

(xxvi) Bestimmen der Summe $(\bar{z}_p)$ der genannten Pole für den Zeitpunkt $n$ als:

(a)

$$\bar{z}_p = \sum_{i=1}^{i=8} z_{i,p}$$

;

oder

$$\bar{z}_p = a_1^{(n)}$$

(b) , wobei $a_1^{(n)}$ der erste autoregressive Koeffizient der genannten zeitvarianten ARMA-Signaldarstellung mit der autoregressiven Ordnung 8 und MA-Ordnung 5 zum Zeitpunkt $n$ ist;

(xxvii) Bestimmen des mittleren Pols $(\bar{z}_p/8)$ der genannten Segmente der genannten EEG-Signale als Summe der Pole, berechnet in Schritt (xxvi), durch Dividieren durch 8;

(xxviii) Substituieren der in Schritt (xxiii) für jeden Zeitpunkt $n$ bestimmten AR- und MA-Koeffizienten in die erste Gleichung von Schritt (xxii) und Berechnen der Amplitudenwerte der genannten EEG-Segmente für einen normalisierten Weißrauscheingang;

(xxix) Erzeugen eines ARMA-Verstärkungswertes ⟨Y(t)⟩ für jeden Zeitschritt $n$ als quadratischen Mittelwert der Amplitudenwerte für die genannte Signaldarstellung, die in Schritt (xxviii) für den genannten Zeitschritt $n$ bestimmt wurden;

(xxx) Schätzen des Eingangs zum Kortex als das Produkt g(q)P(ω), berechnet mit der Gleichung:

$$g(q)P(\omega) = \frac{\langle \tilde{Y}(t) \rangle}{\langle Y(t) \rangle}$$

wobei $\langle Y(t) \rangle$ die in Schritt (xxix) bestimmte ARMA-Verstärkung ist und $\langle \tilde{Y}(t) \rangle$ die durchschnittliche Signalamplitude eines Teils des EEG-Signals repräsentiert, geschätzt für jeden Zeitpunkt $n$ als quadratischen Mittelwert des EEG-Signals, das unter Verwendung eines Gleitfensters fester Länge erhalten wurde;

(xxxi) Berechnen des Kortexeingangs-(CI)-Maßes als den in Schritt (xxx) erzeugten Eingang zum Kortex; und

(xxxii) Berechnen des Kortexzustands-(CS)-Maßes als den in Schritt (xxvii) erzeugten mittleren Pol.

5. Verfahren nach Anspruch 3 oder 4, wobei der Kortexzustand (CS) so skaliert wird, dass er in einen vordefinierten Bereich fällt.

6. Verfahren nach Anspruch 3, 4 oder 5, wobei der Kortexeingang (CI) so skaliert wird, dass er in einen vordefinierten Bereich fällt.

7. Verfahren nach einem der Ansprüche 1 bis 6, wobei die erzeugten Werte der genannten CS- und CI-Paare grafisch als Punkte in einer Ebene, der CS-CI-Ebene, dargestellt werden, die eine über einen Bereich von CS-Werten definierte horizontale Achse und eine über einen Bereich von CI-Werten definierte vertikale Achse hat.

8. Verfahren nach Anspruch 7, wobei eine heuristische/statistische Diskriminierungsfunktion erstellt wird, um Regionen der CS-CI-Ebene zu definieren, die unterschiedliche Funktionseigenschaften in Bezug auf Hirnaktivität haben.

9. Verfahren nach einem der Ansprüche 1 bis 6, wobei die erzeugten Werte der genannten CS- und CI-Paare als eine interpolierte farbige oder schattierte topografische Karte getrennt grafisch dargestellt werden, deren räumliche Abbildung so berechnet wird, dass sie von den physischen Orten der jeweiligen Orte der Elektroden auf der Kopfhaut des Subjekts abhängig ist.

10. System zur Analyse eines Elektroenzephalogramms, das für den Funktionszustand des Gehirns eines Subjekts repräsentativ ist, wobei das Gehirn einen Kortex hat und kortikalen und subkortikalen Eingängen unterliegt, wobei das System Folgendes umfasst:

mehrere Elektroden (102) zum Positionieren auf der Kopfhaut des Subjekts zum Erzeugen von Elektroenzephalogramm-(EEG-)Signalen;
ein Prozessormodul (108); und
Anzeigemittel (104),
wobei das genannte Prozessormodul (108) so gekoppelt ist, dass es die EEG-Signale von den Elektroden empfängt, und so gestaltet ist, dass es:

(i) mehrere EEG-Segmente anhand der EEG-Signale erzeugt;
(ii) die genannten EEG-Segmente als eine ARMA-(Autoregressive Moving Average)-Signaldarstellung fester Ordnung mit einer autoregressiven Ordnung (AR) zwischen 8 und 14 und einer MA-(Moving Average)-Ordnung zwischen 5 und 11 repräsentiert;
(iii) AR- und MA-Koeffizientendaten der genannten ARMA-Signaldarstellung für die genannten EEG-Segmente erzeugt;
(iv) anhand der genannten AR- und MA-Koeffizientendaten Kortexzustands-(CS)-Daten erzeugt, die den Funktionszustand des genannten Kortex zu Zeiten darstellen, die den jeweiligen EEG-Segmenten entsprechen;
(v) Kortexeingangs-(CI)-Daten anhand der AR- und MA-Koeffizientendaten und der genannten EEG-Segmente erzeugt, die den Level an subkortikalen und subkortikalen Eingängen zum genannten Kortex zu Zeiten repräsentieren, die jeweiligen EEG-Segmenten entsprechen;
(vi) anhand der in den Schritten (iv) und (v) erzeugten genannten Kortexzustands-(CS)-Daten und genannten Kortexeingangs-(CI)-Daten Anzeigedaten in Form einer Sequenz von CS- und CI-Paaren zu den gleichen Zeitpunkten wie die entsprechenden EEG-Segmente erzeugt; und wobei das Anzeigemittel (104) zum Anzeigen der Anzeigedaten betreibbar ist.

11. System nach Anspruch 10, wobei das Prozessormodul (108) die folgende Aufgabe hat:

(vii) Geben, dem Anwender des Verfahrens, einer Option zur Auswahl einer zeitvarianten oder zeitinvarianten ARMA-Signaldarstellung in Schritt (iii).

12. System nach Anspruch 11, wobei das Prozessormodul (108) die folgenden Aufgaben hat:

(viii) Auswählen einer zeitinvarianten ARMA-Signaldarstellung fester Ordnung mit der autoregressiven Ordnung 8 und der MA-Ordnung 5, um jedes der EEG-Segmente durch eine erste Gleichung zu beschreiben:

$$y[n] = -\sum_{k=1}^{8} a_k y[n-k] + \sum_{k=0}^{5} b_k u[n-k]$$

wobei $y[n]$ eine ordinale Sequenz von Samples des genannten EEG-Signals des genannten Segments repräsentiert, $y[n-k]$ den $k$-ten vorher abgetasteten Wert von $y[n]$ repräsentiert; $u[n-k]$ einen Gaußschen Weißrauschprozess repräsentiert; und $a_k$ und $b_k$ die geschätzten autoregressiven AR- und MA-(Moving Average)-Koeffizienten repräsentieren, die in Schritt (iii) jeweils für ein genanntes Segment des genannten EEG-Signals erzeugt wurden;

(ix) Lösen der ersten Gleichung zur Bestimmung der AR-Koeffizienten $a_1$ - $a_8$ und der *MA-Koeffizienten* $b_0$ - $b_5$;

(x) Durchführen einer z-Transformation an der ersten Gleichung, um eine z-Domänendarstellung zu erhalten, die durch eine zweite Gleichung angegeben wird:

$$Y(z) = \frac{\sum_{k=0}^{5} b_k z^{-k}}{\sum_{k=0}^{8} a_k z^{-k}} U(z)$$

(xi) Substituieren jedes der in Schritt (ix) erhaltenen Werte der Koeffizienten in die zweite Gleichung von Schritt (x);

(xii) Bestimmen der Pole der zweiten Gleichung von Schritt (x) mittels der folgenden Gleichung:

$$\sum_{k=0}^{8} a_k p^{-k} = \sum_{k=0}^{8} a_k p^{8-k} = 0$$

wobei es 8 Pole gibt, die jeweils durch das Symbol $p$ repräsentiert werden; und

(xiii) Berechnen der Summe der in Schritt (xii) bestimmten Pole, unter Verwendung von:

(a)

$$\bar{z}_p \equiv \sum_{i=1}^{i=8} z_{i,p}$$

,

oder

(b) $\bar{z}_p = a_1$, wobei $a_1$ der erste autoregressive Koeffizient der genannten zeitinvarianten ARMA-Signaldarstellung ist;

(xiv) Bestimmen des mittleren Pols ($\bar{z}_p$/8) der genannten EEG-Segmente als Summe der Pole, berechnet in Schritt (xiii), durch Dividieren durch 8;

(xv) Substituieren der in Schritt (ix) bestimmten AR- und MA-Koeffizienten in die erste Gleichung in Schritt (viii) und Berechnen der Amplitudenwerte der genannten EEG-Segmente für einen normalisierten Weißrauscheingang;

(xvi) Erzeugen eines ARMA-Verstärkungswertes $\langle Y(t) \rangle$ als quadratischen Mittelwert der in Schritt (xv) bestimmten Amplitudenwerte für die genannten Signaldarstellungen;

(xvii) Schätzen des Eingangs zum Kortex als das Produkt $g(q)P(\omega)$

$$g(q)P(\omega) = \frac{\langle \tilde{Y}(t) \rangle}{\langle Y(t) \rangle}$$

wobei $\langle Y(t) \rangle$ die in Schritt (xvii) oben bestimmte ARMA-Verstärkung ist und $\langle \tilde{Y}(t) \rangle$ der quadratische Mittelwert der Amplitude der genannten EEG-Segmente ist;
(xviii) Berechnen des Kortexeingangs-(CI)-Maßes als den in Schritt (xvii) erzeugten Eingang zum Kortex; und
(xix) Berechnen des Kortex Zustands-(CS)-Maßes als den in Schritt (xiv) erzeugten mittleren Pol.

**13.** System nach Anspruch 11, wobei das Prozessormodul die folgenden Aufgaben hat:

(xx) Auswählen einer zeitvarianten ARMA-Signaldarstellung fester Ordnung mit der autoregressiven Ordnung 8 und der MA-Ordnung 5, um jedes der EEG-Segmente durch eine erste Gleichung zu beschreiben:

$$y[n] = -\sum_{k=1}^{8} a_k^{(n)} y[n-k] + \sum_{k=0}^{5} b_k^{(n)} u[n-k]$$

wobei $a_k^{(n)}$ und $b_k^{(n)}$ die geschätzten autoregressiven und MA-Koeffizienten repräsentieren, die in Schritt (iii) jeweils für ein genanntes Segment des genannten EEG-Signals für den Zeitpunkt $n$ erzeugt wurden;
(xxi) rekursives Schätzen der zeitvarianten autoregressiven Koeffizienten $a_1^{(n)}$-$a_8^{(n)}$ und der zeitvarianten MA-Koeffizienten $b_0^{(n)}$-$b_5^{(n)}$ für den Zeitpunkt $n$;
(xxii) Durchführen einer z-Transformation an der genannten ersten Gleichung, um eine z-Domänendarstellung zu erhalten, die durch eine zweite Gleichung angegeben wird:

$$Y(z) = \frac{\sum_{k=0}^{5} b_k z^{-k}}{\sum_{k=0}^{8} a_k z^{-k}} U(z)$$

wobei $Y(z)$ eine ARMA-Darstellung eines Teils des EEG-Signals in der z-Domäne repräsentiert und $U(z)$ einen Gaußschen Weißrauschprozess in der z-Domäne repräsentiert;
(xxiii) Bestimmen der 8 Pole für den Zeitpunkt $n$ als Lösungen für die genannte zweite Gleichung;
(xxiv) Bestimmen der Summe ($\bar{z}_p$) der genannten Pole für den Zeitpunkt $n$ als:

(a)

$$\bar{z}_p \equiv \sum_{i=1}^{i=8} z_{i,p} \; ;$$

oder

$$\bar{z}_p = a_1^{(n)}$$

(b) ; wobei $a_1^{(n)}$ der erste autoregressive Koeffizient der genannten zeitvarianten ARMA-Signaldarstellung mit der autoregressiven Ordnung 8 und der MA-Ordnung 5 zum Zeitpunkt $n$ ist;

(xxv) Bestimmen des mittleren Pols ($\bar{z}_p/8$) der genannten Segmente der genannten EEG-Signale als Summe der Pole, berechnet in Schritt (xxiv), durch Dividieren durch 8;
(xxvi) Substituieren der in Schritt (xxi) für jeden Zeitpunkt $n$ bestimmten AR- und MA-Koeffizienten in die erste Gleichung von Schritt (xx) und Berechnen der Amplitudenwerte der genannten EEG-Segmente für einen normalisierten Weißrauscheingang;

(xxvii) Erzeugen eines ARMA-Verstärkungswertes $\langle Y(t) \rangle$ für jeden Zeitschritt $n$ als quadratischen Mittelwert der Amplitudenwerte für die genannte Signaldarstellung wie in Schritt (xxvi) für den genannten Zeitschritt $n$ bestimmt;

(xxviii) Schätzen des Eingangs zum Kortex als das Produkt $g(q)P(\omega)$, berechnet mit der Gleichung:

$$g(q)P(\omega) = \frac{\langle \tilde{Y}(t) \rangle}{\langle Y(t) \rangle}$$

wobei $\langle Y(t) \rangle$ die in Schritt (xxvii) bestimmte ARMA-Verstärkung ist und $\langle \tilde{Y}(t) \rangle$ die durchschnittliche Signalamplitude eines Teils des EEG-Signals repräsentiert, geschätzt für jeden Zeitpunkt $n$ als quadratischen Mittelwert des EEG-Signals, das unter Verwendung eines Gleitfensters fester Länge erhalten wurde;

(xxix) Berechnen des Kortexeingangs-(CI)-Maßes als den in Schritt (xxviii) erzeugten Eingang zum Kortex; und

(xxx) Berechnen des Kortexzustands-(CS)-Maßes als den in Schritt (xxv) erzeugten mittleren Pol.

14. Computerausführbarer Code, der auf einem computerlesbaren Medium gespeichert ist und, wenn er vom Prozessormodul des Systems gemäß Definition in einem der Ansprüche 10 bis 13 ausgeführt wird, bewirkt, dass das Prozessormodul die Schritte des Verfahrens nach einem Ansprüche 1 bis 9 ausführt.

## Revendications

1. Un procédé d'analyse d'un électroencéphalogramme représentatif de l'état fonctionnel du cerveau d'un sujet, le cerveau possédant un cortex et étant soumis à des entrées corticales et sous-corticales, le procédé comprenant les opérations suivantes :

(i) l'obtention de signaux d'électroencéphalogramme (EEG) à partir d'une pluralité d'électrodes positionnées sur le cuir chevelu du sujet,

(ii) la génération d'une pluralité de segments EEG à partir des signaux EEG,

(iii) la représentation desdits segments EEG sous la forme d'une représentation de signal de moyenne mobile autorégressive à ordre fixe (ARMA) avec un ordre autorégressif (AR) entre 8 et 14 et un ordre de moyenne mobile (MA) entre 5 et 11,

(iv) la génération de données de coefficients AR et MA de ladite représentation de signal ARMA pour lesdits segments EEG,

(v) la génération, en fonction desdites données de coefficients AR et MA, de données d'état cortical (CS) représentant l'état fonctionnel dudit cortex à des instants correspondant à des segments EEG respectifs,

(vi) la génération de données d'entrées corticales (CI) à partir desdites données de coefficients AR et MA et desdits segments EEG représentatifs du niveau d'entrées corticales et sous-corticales vers ledit cortex à des instants correspondant à des segments EEG respectifs,

(vii) la génération, en fonction desdites données d'état cortical (CS) et desdites données d'entrées corticales (CI) générées aux opérations (v) et (vi), de données d'affichage sous la forme d'une séquence de paires CS et CI aux mêmes points temporels que les segments EEG correspondants, et

(viii) l'affichage desdites données d'affichage sur un moyen d'affichage.

2. Un procédé selon la Revendication 1 comprenant l'opération suivante :
(ix) la fourniture à l'utilisateur du procédé d'une option de sélection d'une représentation de signal ARMA variant dans le temps ou invariable dans le temps à l'opération (iv).

3. Un procédé selon la Revendication 2 comprenant les opérations suivantes :

(x) la sélection d'une représentation de signal ARMA invariable dans le temps à ordre fixe d'ordre autorégressif de 8 et d'ordre de moyenne mobile de 5 de façon à décrire chacun des segments EEG par une première équation :

$$y[n] = -\sum_{k=1}^{8} a_k y[n-k] + \sum_{k=0}^{5} b_k u[n-k]$$

où $y[n]$ représente une séquence ordinale d'échantillons dudit signal EEG dudit segment, $y[n-k]$ représente la $k$-ième valeur échantillonnée antérieure de $y[n]$; $u[n-k]$ représente un procédé de bruit blanc gaussien et $a_k$ et $b_k$ représentent les coefficients de moyenne mobile MA et autorégressive AR estimés générés à l'opération (iv) respectivement pour un dit segment dudit signal EEG,

(xi) la résolution de la première équation de façon à déterminer les coefficients AR $a_1$ - $a_8$ et les coefficients MA $b_0$ - $b_5$,

(xii) l'exécution d'une transformée z sur la première équation de façon à obtenir une représentation de domaine z donnée par une deuxième équation,

$$Y(z) = \frac{\sum_{k=0}^{5} b_k z^{-k}}{\sum_{k=0}^{8} a_k z^{-k}} U(z)$$

où Y(z) représente une représentation ARMA de chaque partie du signal EEG dans le domaine z et U(z) représente un procédé de bruit blanc gaussien dans le domaine z,

(xiii) la substitution de chacune des valeurs des coefficients obtenus à l'opération (xi) dans la deuxième équation de l'opération (xii),

(xiv) la détermination de pôles de la deuxième équation de l'opération (xii) au moyen de l'équation :

$$\sum_{k=0}^{8} a_k p^{-k} = \sum_{k=0}^{8} a_k p^{8-k} = 0$$

où il y a 8 pôles, chacun d'eux représenté par le symbole $p$, et

(xv) le calcul de la somme des pôles déterminés à l'opération (xiv) au moyen soit de :

(a)

$$\overline{z}_p \equiv \sum_{i=1}^{i=8} z_{i,p}$$

;

ou de

(b) $\overline{z}_p = a_1$ où $a_1$ est le premier coefficient autorégressif de ladite représentation de signal ARMA invariante dans le temps et où $z_{i,p}$ représente le i-ème pôle,

(xvi) la détermination du pôle moyen ($\overline{z}_p/8$) desdits segments EEG sous la forme de la somme des pôles calculée à l'opération (xv), par une division par 8,

(xvii) la substitution des coefficients MA et AR déterminés à l'opération (xi) dans la première équation à l'opération (x) et le calcul de valeurs d'amplitude desdits segments EEG pour une entrée de bruit blanc normalisée,

(xviii) la génération d'une valeur de gain ARMA $\langle Y(t) \rangle$ sous la forme de la valeur de moyenne quadratique des valeurs d'amplitude pour lesdites représentations de signal déterminées à l'opération (xvii),

(xix) l'estimation de l'entrée vers le cortex sous la forme du produit $g(q)P(\omega)$

$$g(q)P(\omega) = \frac{\langle \tilde{Y}(t) \rangle}{\langle Y(t) \rangle}$$

où $\langle Y(t) \rangle$ est le gain ARMA déterminé à l'opération (xvii) ci-dessus et $\langle \tilde{Y}(t) \rangle$ est la moyenne quadratique de l'amplitude desdits segments EEG,

(xx) le calcul de la mesure de l'entrée corticale (CI) sous la forme de l'entrée vers le cortex générée à l'opération (xix), et

(xxi) le calcul de la mesure de l'état cortical (CS) sous la forme du pôle moyen généré à l'opération (xvi).

4. Un procédé selon la Revendication 2 comprenant les opérations suivantes :

(xxii) la sélection d'une représentation de signal ARMA variant dans le temps à ordre fixe d'ordre autorégressif de 8 et d'ordre de moyenne mobile de 5 de façon à décrire chacun des segments EEG par une première équation :

$$y[n] = -\sum_{k=1}^{8} a_k^{(n)} y[n-k] + \sum_{k=0}^{5} b_k^{(n)} u[n-k]$$

où $a_k^{(n)}$ et $b_k^{(n)}$ représentent les coefficients de moyenne mobile et autorégressifs estimés générés à l'opération (iv) respectivement pour un dit segment dudit signal EEG pour l'instant $n$,

(xxiii) l'estimation récursive des coefficients autorégressifs variant dans le temps $a_1^{(n)}$ - $a_8^{(n)}$ et les coefficients de moyenne mobile variant dans le temps $b_0^{(n)}$ - $b_5^{(n)}$ pour l'instant $n$,

(xxiv) l'exécution d'un transformée z sur ladite première équation de façon à obtenir une représentation de domaine z donnée par une deuxième équation :

$$Y(z) = \frac{\sum_{k=0}^{5} b_k z^{-k}}{\sum_{k=0}^{8} a_k z^{-k}} U(z)$$

où $Y(z)$ représente une représentation ARMA d'une partie du signal EEG dans le domaine z et $U(z)$ représente un procédé de bruit blanc gaussien dans le domaine z,

(xxv) la détermination des 8 pôles pour l'instant $n$ sous la forme de solutions à ladite deuxième équation,

(xxvi) la détermination de la somme $(\bar{z}_p)$ desdits pôles pour l'instant $n$ sous la forme soit de :

(a)

$$\bar{z}_p \equiv \sum_{i=1}^{i=8} z_{i,p}$$

,

ou de

(b) $\bar{z}_p = a_1^{(n)}$ où $a_1^{(n)}$ est le premier coefficient autorégressif de ladite représentation de signal ARMA variant dans le temps d'ordre autorégressif de 8 et d'ordre de moyenne mobile de 5 à l'instant $n$,

(xxvii) la détermination du pôle moyen $(\bar{z}_p/8)$ desdits segments desdits signaux EEG sous la forme de la somme des pôles calculés à l'opération (xxvi), par une division par 8,

(xxviii) la substitution des coefficients MA et AR déterminés à l'opération (xxiii) pour chaque instant $n$ dans la première équation de l'opération (xxii) et le calcul de valeurs d'amplitude desdits segments EEG pour une entrée de bruit blanc normalisée,

(xxix) la génération d'une valeur de gain ARMA $\langle Y(t) \rangle$ pour chaque pas temporel $n$ sous la forme de la valeur de moyenne quadratique des valeurs d'amplitude pour ladite représentation de signal déterminée à l'opération (xxviii) pour ledit pas temporel $n$,

(xxx) l'estimation de l'entrée vers le cortex sous la forme du produit $g(q)P(\omega)$ calculé au moyen de l'équation :

$$g(q)P(\omega) = \frac{\langle \tilde{Y}(t) \rangle}{\langle Y(t) \rangle}$$

où $\langle Y(t) \rangle$ est le gain ARMA déterminé à l'opération (xxix) et où $\langle \tilde{Y}(t) \rangle$ représente l'amplitude de signal moyenne d'une partie du signal EEG estimé pour chaque instant *n* sous la forme de la moyenne quadratique du signal EEG obtenu par l'utilisation d'une fenêtre coulissante de longueur fixe,

(xxxi) le calcul de la mesure de l'entrée corticale (CI) sous la forme de l'entrée vers le cortex générée à l'opération (xxx), et

(xxxii) le calcul de la mesure de l'état cortical (CS) sous la forme du pôle moyen généré à l'opération (xxvii).

5. Un procédé selon la Revendication 3 ou 4 où l'état cortical (CS) est étalonné de façon à se situer à l'intérieur d'une plage prédéfinie.

6. Un procédé selon la Revendication 3, 4 ou 5 où l'entrée corticale (CI) est étalonnée de façon à se situer à l'intérieur d'une plage prédéfinie.

7. Un procédé selon l'une quelconque des Revendications 1 à 6 où les valeurs générées desdites paires CS et CI sont représentées graphiquement sous la forme de points dans un plan, le plan CS-CI, possédant un axe horizontal défini sur une plage de valeurs CS et un axe vertical défini sur une plage de valeurs CI.

8. Un procédé selon la Revendication 7 où une fonction de discrimination heuristique/statistique est construite de façon à définir des zones du plan CS-CI possédant des propriétés fonctionnelles différentes par rapport à une activité cérébrale.

9. Un procédé selon l'une quelconque des Revendications 1 à 6 où les valeurs générées desdites paires CS et CI sont représentées graphiquement séparément sous la forme d'une carte topographique ombrée ou colorée interpolée, dont le mappage spatial est calculé de façon à dépendre des emplacements physiques des emplacements respectifs des électrodes sur le cuir chevelu du sujet.

10. Un système d'analyse d'un électroencéphalogramme représentatif de l'état fonctionnel d'un cerveau d'un sujet, le cerveau possédant un cortex et étant soumis à des entrées corticales et sous-corticales, le système comprenant :

   une pluralité d'électrodes (102) destinées à un positionnement sur le cuir chevelu du sujet pour la génération de signaux d'électroencéphalogramme (EEG),
   un module processeur (108), et
   un moyen d'affichage (104),
   ledit module processeur (108) étant couplé de façon à recevoir les signaux EEG à partir des électrodes et adapté de façon à :

   (i) générer une pluralité de segments EEG à partir des signaux EEG,
   (ii) représenter lesdits segments EEG sous la forme d'une représentation de signal de moyenne mobile autorégressive à ordre fixe (ARMA) avec un ordre autorégressif (AR) entre 8 et 14 et un ordre de moyenne mobile (MA) entre 5 et 11,
   (iii) générer des données de coefficients AR et MA de ladite représentation de signal ARMA pour lesdits segments EEG,
   (iv) générer, en fonction desdites données de coefficients AR et MA, des données d'état cortical (CS) représentant l'état fonctionnel dudit cortex à des instants correspondant à des segments EEG respectifs,
   (v) générer des données d'entrées corticales (CI) à partir desdites données de coefficients AR et MA et desdits segments EEG représentatifs du niveau d'entrées sous-corticales et sous-corticales vers ledit cortex à des instants correspondant à des segments EEG respectifs,
   (vi) générer, en fonction desdites données d'état cortical (CS) et desdites données d'entrées corticales (CI) générées aux opérations (iv) et (v), des données d'affichage sous la forme d'une séquence de paires CS et CI aux mêmes points temporels que les segments EEG correspondants, et où le moyen d'affichage (104) est conçu de façon à afficher les données d'affichage.

11. Un système selon la Revendication 10 où le module processeur (108) est conçu de façon à :

(vii) fournir à l'utilisateur du procédé une option de sélection d'une représentation de signal ARMA variant dans le temps ou invariable dans le temps à l'opération (iii).

**12.** Un système selon la Revendication 11 où le module processeur (108) est conçu de façon à :

(viii) sélectionner une représentation de signal ARMA invariable dans le temps à ordre fixe d'ordre autorégressif de 8 et d'ordre de moyenne mobile de 5 de façon à décrire chacun des segments EEG par une première équation :

$$y[n] = -\sum_{k=1}^{8} a_k y[n-k] + \sum_{k=0}^{5} b_k u[n-k]$$

où $y[n]$ représente une séquence ordinale d'échantillons dudit signal EEG dudit segment, $y[n-k]$ représente la $k$-ième valeur échantillonnée antérieure de $y[n]$; $u[n-k]$ représente un procédé de bruit blanc gaussien et $a_k$ et $b_k$ représentent les coefficients de moyenne mobile MA et autorégressive AR estimés générés à l'opération (iii) respectivement pour un dit segment dudit signal EEG,

(ix) résoudre la première équation de façon à déterminer les coefficients AR $a_1$ - $a_8$ et les coefficients MA $b_0$ - $b_5$,

(x) exécuter une transformée z sur la première équation de façon à obtenir une représentation de domaine z donnée par une deuxième équation :

$$Y(z) = \frac{\sum_{k=0}^{5} b_k z^{-k}}{\sum_{k=0}^{8} a_k z^{-k}} U(z)$$

(xi) substituer chacune des valeurs des coefficients obtenus à l'opération (ix) dans la deuxième équation de l'opération (x),

(xii) déterminer les pôles de la deuxième équation de l'opération (x) au moyen de l'équation :

$$\sum_{k=0}^{8} a_k p^{-k} = \sum_{k=0}^{8} a_k p^{8-k} = 0$$

où il y a 8 pôles, chacun d'eux étant représenté par le symbole $p$, et

(xiii) calculer la somme des pôles déterminés à l'opération (xii) au moyen soit de:

(a)

$$\overline{z}_p \equiv \sum_{i=1}^{i=8} z_{i,p}$$
,

ou de

(b) $\overline{z}_p = a_1$ où $a_1$ est le premier coefficient autorégressif de ladite représentation de signal ARMA invariable dans le temps.

(xiv) déterminer le pôle moyen ($\overline{z}_p/8$) desdits segments EEG sous la forme de la somme des pôles calculés à l'opération (xiii), par une division par 8,

(xv) substituer les coefficients MA et AR déterminés à l'opération (ix) dans la première équation à l'opération (viii) et calculer des valeurs d'amplitude desdits segments EEG pour une entrée de bruit blanc normalisée,

(xvi) générer une valeur de gain ARMA $\langle Y(t) \rangle$ sous la forme de la valeur de moyenne quadratique des valeurs d'amplitude pour lesdites représentations de signal déterminées à l'opération (xv),

(xvii) estimer l'entrée vers le cortex sous la forme du produit $g(q)P(\omega)$

$$g(q)P(\omega) = \frac{\langle \tilde{Y}(t) \rangle}{\langle Y(t) \rangle}$$

où $\langle Y(t) \rangle$ est le gain ARMA déterminé à l'opération (xvii) ci-dessus et $\langle \tilde{Y}(t) \rangle$ est la moyenne quadratique de l'amplitude desdits segments EEG,

(xviii) calculer la mesure de l'entrée corticale (CI) sous la forme de l'entrée vers le cortex générée à l'opération (xvii), et

(xix) calculer la mesure de l'état cortical (CS) sous la forme du pôle moyen généré à l'opération (xiv).

**13.** Un système selon la Revendication 11 où le module processeur est conçu de façon à :

(xx) sélectionner une représentation de signal ARMA variant dans le temps à ordre fixe d'ordre autorégressif de 8 et d'ordre de moyenne mobile de 5 de façon à décrire chacun des segments EEG par une première équation :

$$y[n] = -\sum_{k=1}^{8} a_k^{(n)} y[n-k] + \sum_{k=0}^{5} b_k^{(n)} u[n-k]$$

où $a_k^{(n)}$ et $b_k^{(n)}$ représentent les coefficients de moyenne mobile et autorégressifs estimés générés à l'opération (iii) respectivement pour un dit segment dudit signal EEG pour l'instant $n$,

(xxi) estimer récursivement les coefficients autorégressifs variant dans le temps $a_1^{(n)}$ - $a_8^{(n)}$ et les coefficients de moyenne mobile variant dans le temps $b_0^{(n)}$ - $b_5^{(n)}$ pour l'instant $n$,

(xxii) exécuter une transformée z sur ladite première équation de façon à obtenir une représentation de domaine z donnée par une deuxième équation :

$$Y(z) = \frac{\sum_{k=0}^{5} b_k z^{-k}}{\sum_{k=0}^{8} a_k z^{-k}} U(z)$$

où $Y(z)$ représente une représentation ARMA d'une partie du signal EEG dans le domaine z et $U(z)$ représente un procédé de bruit blanc gaussien dans le domaine z,

(xxiii) déterminer les 8 pôles pour l'instant $n$ sous la forme de solutions à ladite deuxième équation,

(xxiv) déterminer la somme $(\bar{z}_p)$ desdits pôles pour l'instant $n$ sous la forme soit de :

(a)

$$\bar{z}_p = \sum_{i=1}^{i=8} z_{i,p}$$

,

ou de

(b) $\quad \bar{z}_p = a_1^{(n)} \quad$ où $a_1^{(n)}$ est le premier coefficient autorégressif de ladite représentation de signal ARMA variant dans le temps d'ordre autorégressif de 8 et d'ordre de moyenne mobile de 5 à l'instant $n$,

(xxv) déterminer le pôle moyen $(\bar{z}_p/8)$ desdits segments desdits signaux EEG sous la forme de la somme des pôles calculés à l'opération (xxiv), par une division par 8,

(xxvi) substituer les coefficients MA et AR déterminés à l'opération (xxi) pour chaque instant $n$ dans la première équation de l'opération (xx) et calculer des valeurs d'amplitude desdits segments EEG pour une entrée de bruit blanc normalisée,

(xxvii) générer une valeur de gain ARMA $\langle Y(t) \rangle$ pour chaque pas temporel $n$ sous la forme de la valeur de moyenne quadratique des valeurs d'amplitude pour ladite représentation de signal déterminée à l'opération (xxvi) pour ledit pas temporel $n$,

(xxviii) estimer l'entrée vers le cortex sous la forme du produit $g(q)P(\omega)$ calculé au moyen de l'équation :

$$g(q)P(\omega) = \frac{\langle \tilde{Y}(t) \rangle}{\langle Y(t) \rangle}$$

où $\langle Y(t) \rangle$ est le gain ARMA déterminé à l'opération (xxvii) et où $\langle \tilde{Y}(t) \rangle$ représente l'amplitude de signal moyenne d'une partie du signal EEG estimé pour chaque instant $n$ sous la forme de la moyenne quadratique du signal EEG obtenu par l'utilisation d'une fenêtre coulissante de longueur fixe,

(xxix) calculer la mesure de l'entrée corticale (CI) sous la forme de l'entrée vers le cortex générée à l'opération (xxviii), et

(xxx) calculer la mesure de l'état cortical (CS) sous la forme du pôle moyen généré à l'opération (xxv).

14. Du code exécutable par ordinateur conservé en mémoire sur un support lisible par ordinateur qui, lorsqu'il est exécuté par le module processeur du système selon l'une quelconque des Revendications 10 à 13, amène le module processeur à exécuter les opérations du procédé selon l'une quelconque des Revendications 1 à 9.

**Figure 1**

200

( start )

Receive EEG signal from subject — 202

Amplify EEG signal — 204

Filter EEG signal — 206

Generate digital EEG samples — 208

210
YES ◇ Use time varying ARMA model? ◇ NO

Generate plurality of segments — 216

212
Perform artefact rejection on EEG samples

Perform artefact rejection on segments — 218

214
Generate ARMA coefficients for each window using (8,5) order time varying ARMA model

Generate ARMA coefficients for each segment using (8,5) order time invariant ARMA model — 220

222
Generate ARMA gain data and signal gain data for the ARMA model used to process the digitised EEG signal

Generate pole data, zeros data, mean pole data and index data based on the ARMA coefficients — 224

223
Generate product data based on the ARMA gain data and signal gain data

Generate display data (and/or output data) based on the mean pole data and/or product data — 226

( end )

**Figure 2**

300

308    312    314

310

Monitor    Sensor Check    Setup

320 → REALTIME    recording started at    13/04/2005 10:32:18    Stop

302

306 →    μV
+50

57

0

-50

304

316
318

80
60
40
20

54    10:55    10:56    10:57    10:58    10:59    11:00    11:01    11:02    11:03

334

322 → 13/04/2005 11:32:20    Mark Event    Red    Green    Blue

324    326    328

**Figure 3**

300

Monitor    Sensor Check    Setup

310

320 → REALTIME MONITOR STOPPED    Start

μV
+50

57

0

-50

80
60
40
20

54    10:55    10:56    10:57    10:58    10:59    11:00    11:01    11:02    11:03

334

REVIEW    session recorded at    20050413_110513 ▼    Delete File

330    332

**Figure 4**

500

Monitor    Sensor Check    Setup

BarSensor

502    504    506

Sensor Check Rate (Hz) 5

Start Auto Check

Stop Auto Check

Low Pass Filter (Hz) 128

**Figure 5**

600

Monitor    Sensor Check    Setup

602    604    606    608    610

Display    Date Time    DAM    Serial Output    Printer

BAR Colour Thresholds

Set

80

Set

60

Set

40

Set

20

Set

Event Markers

☑ Show markers on BAR chart

List Events

Clear Events Log

refresh (ms) 64

smoothing 5

EEG

Sweep Speed

○ 8 mm/sec

○ 16 mm/sec

◉ 32 mm/sec

Sensitivity

○ 10 µV/div

○ 20 µV/div

◉ 50 µV/div

○ 100 µV/div

**Figure 6**

**Figure 7**

**Figure 8**

EP 2 088 924 B1

Figure 9

Figure 10

38

**Figure 11**

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 200174248 A
- EP 0898234 A
- WO 2004054441 A
- WO 2004064633 A
- EP 1704819 A
- EP 1563789 A
- WO 2007140535 A
- WO 2007140536 A

## Non-patent literature cited in the description

- *Anesth Analg,* 2003, vol. 97, 488-491
- **TARVAINEN et al.** Estimation of non-stationary EEG with Kalman smoother approach: an application to event-related synchronization (ERS). *IEEE Trans Biomed Eng,* 2004, vol. 51, 516-524
- **LJUNG L.** System Identification - Theory for the User. Prentice Hall, 1999
- **HOPKINS PM.** Nitrous oxide: a unique drug of continuing importance for anaesthesia. *Best Pract Res Clin Anaesthesiol.,* September 2005, vol. 19 (3), 381-9
- **RUDOLPH U ; ANTKOWIAK B.** Molecular and neuronal substrates for general anaesthetics. *Nat Rev Neurosci.,* September 2004, vol. 5 (9), 709-20
- **BRENNER RP ; ULRICH RF ; SPIKER DG ; SCLABASSI RJ ; REYNOLDS III CF ; MARIN RS ; BOLLER F.** Computerized EEG spectral analysis in elderly normal, demented and depressed subjects. *Electroencephalogr Clin Neurophysiol,* 1986, vol. 64, 483-92
- **COBEN LA ; DANZIGER W ; STORANDT M.** A longitudinal EEG study of mild senile dementia of Alzheimer type. *Electroencephalogr Clin Neurophysiol,* 1985, vol. 61, 101-12
- **GIAQUINTO S ; NOLFE G.** The EEG in the normal elderly: a contribution to the interpretation of aging and dementia. *Electroencephalogr Clin Neurophysiol,* 1986, vol. 63, 540-6
- **DUNKIN JJ ; LEUCHTER AF ; NEWTON TF ; COOK IA.** Reduced EEG coherence in dementia: state or trait marker?. *Biol Psychiatry,* 1994, vol. 35, 870-9
- **LOCATELLI T ; CURSI M ; LIBERATI D ; FRANCESCHI M ; COMI G.** EEG coherence in Alzheimer's disease. *Electroencephalogr Clin Neurophysiol,* 1998, vol. 106, 229-37
- **JEONG J.** EEG dynamics in patients with Alzheimer's disease. *Clin Neurophysiol,* 2004, vol. 115, 1490-1505
- **FERREE TC.** Spherical splines and average referencing in scalp electroencephalography. *Brain Topogr,* 04 October 2006
- **SRINIVASAN R ; NUNEZ PL ; SILBERSTEIN R.** Spatial filtering and neocortical dynamics: estimates of EEG coherence. *IEEE Trans Biomed Eng,* 1998, vol. 45, 814-26
- **FREEMAN WJ.** Use of spatial deconvolution to compensate for distortion of EEG by volume conduction. *IEEE Trans Biomed Eng,* 1980, vol. 27, 421-9
- **SOONG AC ; LIND JC ; SHAW GR ; KOLES ZJ.** Systematic comparisons of interpolation techniques in topographic brain maping. *Electroencephalogr Clin Neurophysiol,* 1993, vol. 87, 185-95